# FASCICULE DE BREVET EUROPEEN

(11) **EP 2 155 892 B1**
(45) Date de publication et mention de la délivrance du brevet: **18.03.2015**
(21) Numéro de dépôt: 08805880.5
(22) Date de dépôt: 29.05.2008
(51) Int. Cl.: C12Q 1/04, C12Q 1/37, C07D 235/18, C07D 241/44, C07D 263/56, C07D 277/66, C07K 5/06

(54) **NOUVEAUX SUBSTRATS DE PEPTIDASE**
NEUARTIGE PEPTIDASE-SUBSTRATE
NOVEL PEPTIDASE SUBSTRATES

(30) Priorité: 31.05.2007 FR 0755371
(43) Date de publication de la demande: 24.02.2010
(60) Demande divisionnaire: 12162353.2
(73) Titulaire: BIOMERIEUX, 69280 Marcy-L'Etoile (FR)
(72) Inventeur: FABREGA, Olivier, Newcastle Upon Tyne NE2 1NE (GB); JAMES, Arthur, Cumbria CA13 9UX (GB); SALWATURA, Vindhya Lakshika, Newcastle Upon Tyne NE4 9JB (GB); ORENGA, Sylvain, F-01160 Neuville Sur Ain (FR); STANFORTH, Stephen, Northumberland, NE43 7EH (GB)
(86) Numéro de dépôt international: PCT/FR2008/050936
(87) Numéro de publication internationale: WO 2008/152306

(56) Documents cités:
- FR-A- 2 751 663
- US-A- 5 316 906
- US-A1- 2005 272 765
- DIKOV A ET AL.: "New fluorogenic substrates in histochemistry of peptidases: I. Histochemical determination of alanineaminopeptidase" DOKLADI NA BULGARSKATA AKADEMIYA NA NAUKITE, vol. 49, no. 4, 1996, pages 89-92, XP008056562 ISSN: 0861-1459
- DATABASE REGISTRY STN; Different Chemical Libraries Suppliers 15 mars 2007 (2007-03-15), XP002465502
- MANAFI M ET AL: "FLUOROGENIC AND CHROMOGENIC SUBSTRATES USED IN BACTERIAL DIAGNOSTICS", MICROBIOLOGICAL REVIEWS, AMERICAN SOCIETY FOR MICROBIOLOGY, WASHINGTON, DC, US, vol. 55, no. 3, 1 September 1991 (1991-09-01), pages 335-348, XP009020939, ISSN: 0146-0749
- ORENGA SYLVAIN ET AL: "Enzymatic substrates in microbiology", JOURNAL OF MICROBIOLOGICAL METHODS, NETHERLANDS, vol. 79, no. 2, 11 August 2009 (2009-08-11), pages 139-155, XP002562182, DOI: 10.1016/J.MIMET.2009.08.001

## Description

La présente invention concerne de nouveaux substrats enzymatiques pour la détection d'activité peptidase. Ces substrats sont utilisables dans les applications comportant une étape d'hydrolyse enzymatique produisant un signal physico-chimique notamment en microbiologie, biochimie, immunologie, biologie moléculaire, histologie, etc. L'invention concerne également des milieux réactionnels contenant de tels substrats, l'utilisation des substrats ou des milieux pour la détection d'activités peptidases et/ou la différenciation de bactéries à Gram positif par rapport à des bactéries à Gram négatif et des procédés d'utilisation.

Il existe actuellement de très nombreux milieux permettant la détection de microorganismes. Cette détection peut être basée notamment sur l'utilisation de substrats particuliers, spécifiques d'une enzyme du microorganisme que l'on souhaite détecter. D'une manière générale, les substrats synthétiques d'enzymes sont constitués d'une première partie spécifique de l'activité enzymatique à révéler, et d'une seconde partie faisant office de marqueur, généralement chromogène ou fluorescent. Ainsi dans le cas des bactéries, par le choix des substrats, selon qu'il y a réaction ou non, il est possible de caractériser la nature d'un microorganisme. Une activité peptidase peut notamment être utilisée pour révéler un groupe, un genre ou une espèce de bactéries. Ainsi, l'activité alanine-aminopeptidase, par exemple, permet de différencier les bactéries à Gram négatif des bactéries à Gram positif.

Des substrats chromogènes enzymatiques pour la détection d'activité peptidase sont connus de l'état de la technique. On peut citer notamment la publication de Manafi (Manafi et al, Microbiol Rev 55(3) : 335-348, 1991), qui est une revue de substrats enzymatiques utilisés en microbiologie. Toutefois, les substrats d'aminopeptidase décrits libèrent par hydrolyse des composés diffusant dans le milieu (beta-naphtylamine, 7-amino-4-méthylcoumarine). De ce fait, en milieu réactionnel hétérogène (colonies sur boites de Pétri, coupe histologique...), il n'est pas possible de localiser précisément le lieu de l'hydrolyse. On peut citer également les substrats décrits dans les demandes de brevet WO 98/04735 et WO 99/38995 déposées par la Demanderesse. Toutefois, bien que ces substrats diffusent peu en milieu de culture, ils présentent certains inconvénients : leur synthèse est difficile, la pureté est réduite, les rendements faibles et ils sont toxiques vis-à-vis de certains micro-organismes.

La présente invention propose donc de nouveaux substrats de peptidase permettant la détection de microorganismes. Comparativement aux substrats existants, ces nouveaux substrats sont de synthèse aisée, et peuvent être utilisés notamment en milieux gélifiés pour la détection de micro-organismes car ils produisent une coloration ne diffusant pas dans le milieu réactionnel. Cela permet de repérer une colonie ou une organelle exprimant une activité peptidase parmi d'autres ne l'exprimant pas.

Avant d'aller plus avant dans la description de l'invention, les définitions ci dessous sont données afin de faciliter l'exposé de l'invention.

Par substrat enzymatique, on entend un substrat pouvant être hydrolyse par une enzyme en un produit permettant la détection, directe ou indirecte d'un microorganisme, d'une cellule ou d'une organelle. Ce substrat comprend notamment une première partie spécifique de l'activité enzymatique à révéler et une seconde partie faisant office de marqueur.

Les substrats selon l'invention sont particulièrement bien adaptés à une utilisation en cytométrie en flux, car le produit de l'hydrolyse restant principalement localisé dans la cellule exprimant l'activité enzymatique, il est possible de compter spécifiquement les cellules exprimant cette activité, voire de les séparer du reste de l'échantillon.

Les substrats selon l'invention sont également bien adaptés à une utilisation en histoenzymologie, car le produit d'hydrolyse restant principalement localisé sur le lieu de l'hydrolyse, il est possible d'identifier spécifiquement les cellules ou organelles exprimant cette activité au sein d'un tissu.

Du fait de leur faible toxicité, les substrats selon l'invention sont bien adaptés au suivi d'activité peptidase de culture cellulaire.

Les substrats selon l'invention sont également très bien adaptés à une utilisation en milieu de détection et/ou d'identification car ils produisent une coloration ou une fluorescence ne diffusant pas dans le milieu réactionnel. Dans la présente demande, le terme coloration est employé pour couvrir une coloration, absorption de lumière dans le spectre visible, ou une fluorescence, absorption à une longueur d'onde (λₑₓ) et émission à une longueur d'onde supérieure (λ_{em,} λₑₘ > λₑₓ).

Les substrats de l'invention peuvent être salifiés, c'est à dire sous forme de sel tel que chlorure, bromure ou trifluoroacétate.

Par peptidase, on entend une enzyme capable de cliver par hydrolyse le groupement amide formé entre le reste acyle d'un peptide et une amine primaire. Par aminopeptidase, on entend une enzyme capable de cliver par hydrolyse le groupement amide formé entre un acyle d'acide aminé et une amine primaire. Dans la présente demande, le terme « peptidase » peut désigner, selon les cas, tant une peptidase qu'une aminopeptidase telles que définies précédemment.

Par peptide, on entend une chaîne peptidique comprenant de 1 à 10 acides aminés, préférentiellement de 1 à 4 acides aminés. Préférentiellement, le peptide est un di-Alanine ou tri-Alanine. Par acide aminé, on entend tout acide aminé connu de l'homme du métier, naturel ou non. Selon un mode particulier de réalisation de l'invention, l'acide aminé est une beta Alanine ou L Alanine, ou une Glycine, Pyrroglutamyl, .... Ledit peptide peut comprendre un agent de blocage en son extrémité N terminale. Les agents de blocage selon l'invention comprennent tout agent de blocage connu de l'homme du métier qui est capable de protéger les amines. A titre d'exemple, on peut citer le t-Butoxycarbonyle (N-tBOC), le 9-Fluorenyloxycarbonyle, un agent de solubilisation tel que le Succinyle, ou bien un acide aminé non métabolisable, c'est-à-dire non naturel, tel que l'acide pipécolique ou la forme D d'un acide aminé, tel que la D-Phénylalanine. Les agents de blocage ne sont pas systématiquement présents dans les composés de l'invention.

Par groupement alkyle, on entend une chaîne de groupements hydrocarbonés saturés, tel que notamment un alkyle en C₁-C₆, c'est a dire un alkyle droit ou ramifié ayant de 1 à 6 atomes de carbone. A titre d'exemple, on peut citer le méthyle, l'éthyle, le propyle, l'iso-propyle, le butyle, le t-butyle, le pentyle, l'iso-pentyle et l'hexyle.

Par groupement aryle, on entend un groupement fonctionnel (ou substituant) qui dérive d'un noyau aromatique tel que notamment un noyau aromatique en C6-C10, notamment phényle, benzyle, 1-naphtyle ou 2-naphtyle.

Par groupement carboxyl, on entend notamment un groupe fonctionnel composé d'un atome de carbone, lié par une double liaison à un premier atome d'oxygène, et par une liaison simple à un second atome d'oxygène, lui-même chargé négativement ou relié à un atome d'hydrogène. En fonction du pKₐ de la molécule et du pH du milieu, le groupement carboxyle peut être sous forme ionisée, c'est à dire sans H lié au second atome d'oxygène, qui est alors chargé négativement.

Par milieu réactionnel, on entend un milieu comprenant tous les éléments nécessaires à l'expression d'un métabolisme et/ou à la croissance de microorganismes, d'une cellule ou d'une organelle. Ce milieu réactionnel peut être utilisé en cytométrie en flux, histoenzymologie, culture cellulaire... ou en tant que milieu de détection et/ou d'identification de microorganismes.

Le milieu réactionnel peut comprendre un ou plusieurs éléments en combinaison, tels que des acides aminés, des peptones, des hydrates de carbone, des nucléotides, des minéraux, des vitamines, des antibiotiques, des tensioactifs, des tampons, des sels de phosphate, d'ammonium, de sodium, de métaux.

Le milieu peut comprendre également un colorant. A titre indicatif, on peut citer comme colorant le bleu d'Evans, du rouge neutre, du sang de mouton, du sang de cheval, un opacifiant tel que l'oxyde de Titane, de la nitroaniline, du vert malachite, du vert brillant...

Le milieu réactionnel peut être solide, semi-solide ou liquide. Par milieu solide, on entend par exemple un milieu gélifié. L'agar est l'agent gélifiant traditionnel en microbiologie pour la culture des microorganismes, mais il est possible d'utiliser de la gélatine ou de l'agarose. Un certain nombre de préparation sont disponibles dans le commerce, comme par exemple l'agar Columbia, la gélose Trypcase-soja, la gélose Mac Conkey, la gélose Sabouraud ou plus généralement celles décrites dans le Handbook of Microbiological Media (CRC Press).

Le milieu réactionnel peut être un milieu de détection et/ou d'identification, c'est à dire un milieu de révélation ou un milieu de culture et de révélation. Dans le premier cas, la culture des microorganismes est effectuée avant ensemencement et, dans le deuxième cas, le milieu de détection et/ou d'identification constitue également le milieu de culture.

Par échantillon biologique, on entend un échantillon clinique, issu d'un prélèvement de liquide biologique ou un échantillon alimentaire, issu de tout type d'aliment. Cet échantillon peut être ainsi liquide ou solide et on peut citer d'une manière non limitative, un échantillon clinique de sang, de plasma, d'urine, de fécès, de prélèvement de nez, de gorge, de peau, de plaie, de liquide céphalo-rachidien, un échantillon alimentaire d'eau, de boisson tels que le lait, un jus de fruits; de yaourt, de viande, d'oeuf, de légume, de mayonnaise, de fromage ; de poisson..., un échantillon alimentaire issu d'une alimentation destinée aux animaux, tel que notamment un échantillon issu de farine animale. L'échantillon peut également être un prélèvement de l'environnement clinique, d'élevage ou de production alimentaire, cosmétique ou pharmaceutique. Par prélèvement d'environnement, on entend notamment un prélèvement de surface, de liquide, de matière première ou de produit.

Au sens de la présente invention, le terme microorganisme recouvre les bactéries, les levures, et plus généralement, les organismes généralement unicellulaires, invisibles à l'oeil nu, qui peuvent être multipliés et manipulés en laboratoire.

A titre de bactéries à Gram négatif, on peut citer les bactéries des genres suivants : *Pseudomonas, Escherichia, Salmonella, Shigella, Enterobacter, Klebsiella, Serratia, Proteus, Campylobacter, Haemophilus*, *Morganella*, *Vibrio*, *Yersinia, Acinetobacter, Branhamella, Neisseria, Burkholderia, Citrobacter, Hafnia, Edwardsiella, Aeromonas, Moraxella, Pasteurella, Providencia, Actinobacillus, Alcaligenes, Bordetella, Cedecea, Erwinia, Pantoea, Ralstonia, Stenotrophomonas, Xanthomonas* et *Legionella.*

A titre de bactéries à Gram positif, on peut citer les bactéries des genres suivants : *Aerococcus, Enterococcus, Streptococcus, Staphylococcus, Bacillus, Lactobacillus, Listeria, Clostridiarm, Gardnerella, Kocuria, Lactococcus, Leuconostoc, Micrococcus, Falkamia, Gemella, Pediococcurs, Mycobacterium* et *Corynebacterium.*

A titre de levures, on peut citer les levures des genres suivants: *Candida, Cryptococcus, Saccharomyces* et *Trichosporon.*

L'invention concerne un substrat enzymatique pour la détection d'une activité peptidase de formule (I) suivante : 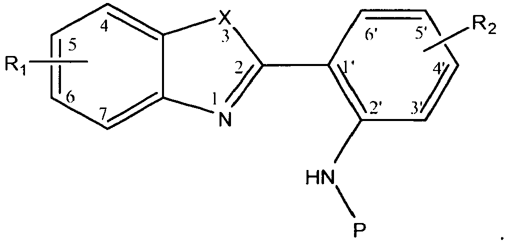 selon laquelle :
□ X représente S;
□ R1 représente rien, ou un des substituants suivants : Cl, Br, F, I, OH, un groupement alkyle, aryle, carboxyle,
□ R2 est rien ou un des substituants suivants : Cl ; O-CH₃ ; F, diéthylènediamine-CH₃, NR3R4, Br, I, OH, un groupement alkyle, aryle, carboxyle, NO₂ 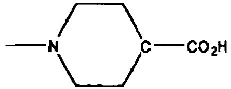 ou O-CH₂-O ; dans ce dernier cas, R2 représente un cycle accolé à l'aminophényl et deux de leurs sommets sont partagés ;
□ X1 est choisi parmi : H, C₂H₄Ph, OH, un groupement alkyle, un groupement aryle
□ R3 et R4 sont indépendamment H ou un groupement alkyle ayant de 1 à 4 atomes de carbone
□ P est une chaine peptidique comprenant de 1 à 10 acides aminés et ne comprenant pas un agent de blocage en son extrémité N-terminale.

La liaison entre l'Azote en 1 et le Carbone en 2 de l'hétérocycle peut être simple (1,2-Dihydro) ou double, préférentiellement double.

Bien entendu, le substrat selon l'invention peut comprendre plusieurs substituants, c'est à dire plusieurs groupements R1, R2 en différente position du cycle. Lorsque R1 et/ou R2 est un groupement alkyle ou aryle, il peut également être substitué par un ou plusieurs substituants tel que OH, carboxyle, Br, Cl, F, I. Lorsque R2 représente O-CH₂-O, R2 représente un cycle accolé à l'aminophényl, deux de leurs sommets étant partagés.

Selon un mode préféré de réalisation de l'invention, X représente S.

Selon un mode particulier de réalisation de l'invention, R1 est en position 5.

Selon un mode particulier de réalisation de l'invention, R2 est en position 4'. Selon un autre mode particulier de réalisation de l'invention, R2 est en position 5'. Selon un autre mode particulier de réalisation de l'invention, R2 est en position 4' et en position 5'.

Selon un mode particulier de réalisation de l'invention, P comprend à son extrémité N-terminale, un agent de blocage.

Selon un l'invention, ledit substrat enzymatique répond à la formule (1) ci dessus et selon laquelle :
- X représente S
- R1 ne représente rien
- R2 ne représente rien
- P représente un peptide, préférentiellement un acide aminé, préférentiellement, choisi parmi L Alanine ou une beta Alanine.

Selon un mode particulier de réalisation de l'invention, ledit substrat enzymatique répond à la formule (I) ci dessus, X représente S, R1 et R2 ne représentent rien et P représente L Alanine. Selon un mode particulier de réalisation de l'invention, ledit substrat enzymatique répond à la formule (1) ci dessus, X représente S, R1 et R2 ne représentent rien et P représente beta Alanine.

Selon un autre mode particulier de réalisation de l'invention, ledit substrat enzymatique répond à la formule (I) ci dessus et selon laquelle :
- X représente S
- R1 ne représente rien
- R2 représente Cl
- P représente un peptide

Préférentiellement, P est un acide aminé, préférentiellement L Alanine. Préférentiellement R2 est en position 5'.

Selon un mode particulier de réalisation de l'invention, ledit substrat enzymatique répond à la formule (I) ci dessus, X représente S, R1 ne représente rien, R2 représente Cl en position 5' et P représente L Alanine.

Selon un mode particulier de réalisation de l'invention, ledit substrat enzymatique répond à la formule (1) telle que définie précédemment et selon laquelle :
- X représente S
- R1 représente CH₃
- R2 ne représente rien
- P représente un peptide, préférentiellement un acide aminé, préférentiellement L Alanine

Selon un mode particulier de réalisation de l'invention, ledit substrat enzymatique répond à la formule (I) ci dessus, X représente S, R1 représente CH₃ en position 5, R2 ne représente rien, et P est L Alanine.

Préférentiellement, le substrat selon l'invention est choisi parmi les substrats décrits dans le tableau 1 avec X=S.

**Tableau 1 - Exemples de substrats selon l'invention**

| **Substrats** | **Référence** | **X** | **X1** | **R1** | **R2** | **P** |
|---|---|---|---|---|---|---|
| 2-(L-Alanvl-2'-amidophenyl)-benzoxazole | Substrat 1 VLS206, 27 | O | - | - | - | L-Ala |
| 2-(β-Alanyl-2'-amidophényl)-benzoxazole | Substrat 2 OF22A | O | - | - | - | β-Ala |
| 2-(Glycyl-2'-amidophényl)-benzoxazole | Substrat 3 OF30B | O | - | - | - | Gly |
| 2-(2'-L-Alanyl-amidophényl)-5-chloro-benzoxazole | Substrat 4 35 | O | - | Cl | - | L-Ala |
| 2-(L-Alanyl-2'-amidophényl)-5-méthylbenzoxazole | Substrat 5 | O | - | CH₃ | - | L-Ala |
| 2-(L-Alanyl-2'-amido-5'-chloro-phényl)-benzoxazole | Substrat 6 (VLS283 | O | - | - | Cl | L-Ala |
| 2-(β-Alanyl-2'-amido-5'-chloro-phényl)-benzoxazole | Substrat 7 OF23A | O | - | - | Cl | β-Ala |
| 2-(L-Alanyl-2'-amido-4',5'-cyclométhylènedioxy-phényl)-benzoxazole | Substrat 8 | O | - | - | O-CH₂-O | L-Ala |
| 2-(L-Alanyl-2'-amido-5'-chloro-phényl)-benzoxazole | Substrat 6 VLS283 | O | - | - | Cl | L-Ala |
| 2-(β-Alanyl-2'-amido-5'-chloro-phényl)-benzoxazole | Substrat 7 OF23A | O | - | - | Cl | ß-Ala |
| 2-(L-Alanyl-2'-amido-4',5'-cyclométhylènedioxy-phényl)-benzoxazole | Substrat 8 | O | - | - | O-CH₂-O | L-Ala |
| 2-(ß-Alanyl-2'-amido-4',5'-cyclométhylènedioxy-phényl)-benzoxazole | Substrat 9 | O | - | - | O-CH₂-O | ß-Ala |
| 2-(L-Alanyl-2'-amido-4',5'-diméthoxyphényl)-benzoxazole | Substrat 10 VLS284 | O | - | - | O-CH₃, O-CH₃ | L-Ala |
| 2-(β-Alanyl-2'-amido-4',5'-diméthoxyphényl)-benzoxazole | Substrat 11 OF24A | O | - | - | O-CH3, O-CH₃ | ß-Ala |
| 2-(β-Alanyl-2'-amido-5'-fluoro-phényl)-benzoxazole | Substrat 12 OF28A | O | - | - | F | ß-Ala |
| 2-(L-Alanyl-2'-amidophényl)-3-méthyl-benzimidazole | Substrat 13 43 | NX1 | CH₃ | - | - | L-Ala |
| 2-(L-Alanyl-2'-amidophényl)-3-phényléthylbenzimidazole | Substrat 14 57 | NX1 | C₂H₄Ph | - | - | L-Ala |
| 2-(L-Alanyl-2'-amido-5'-chloro-phényl)-3-phenyléthyl-benzimidazole | Substrat 15 59 | NX1 | C₂H₄Ph | - | Cl | L-Ala |
| 2-(L-Alanyl-2'-amido-4',5'-méthylènedioxyphényl)-3-phényléthyl-benzimidazole | Substrat 16 60 | NX1 | C₂H₄Ph | - | O-CH₂-O | L-Ala |
| 2-(L-Alanyl-2'-amidophényl)-benzothiazole | Substrat 17 OF38A | S | - | - | - | L-Ala |
| 2-(β-Alanyl-2'-amidophényl)-benzothiazole | Substrat 18 OF33A | S | - | - | - | β-Ala |
| 2-(L-Alanyl-2'-amido-5'-chloro-phényl)-benzothiazole | Substrat 19 | S | - | - | Cl | L-Ala |
| 2-(L-Alanyl-2'-amidophényl)-5-méthylbenzothiazole | Substrat 20 | S | - | CH₃ | - | L-Ala |
| 2-(L-Alanyl-2'-amido-5'-chloro-phényl)-quinoxazol-4-one | Substrat 21 VLS289 | NX1-CO | H | - | Cl | L-Ala |
| 2-(L-Alanyl-2'-amidophényl)-3-hydroquinazolin-4-one | Substrat 22 VLS298 | NX1-CO | H | | | L-Ala |
| 2-(L-Alanyl-2'-amidophényl)-3-méthylquinazoline-4-one | Substrat 23 VLS297 | NX1-CO | CH₃ | | | L-Ala |

L'invention concerne également un milieu réactionnel comprenant au moins un substrat enzymatique tel que défini ci avant.

Préférentiellement, ledit milieu réactionnel est un milieu de détection et/ou d'identification de microorganismes, ledit milieu comprenant au moins un substrat enzymatique tel que défini ci avant.

Préférentiellement, ledit substrat est à une concentration comprise entre 1 et 1000 mg/l, préférentiellement entre 10 et 500 mg/l.

Selon un mode particulier de réalisation de l'invention, ledit milieu de détection et/ou d'identification selon l'invention comprend en outre au moins un autre substrat enzymatique, spécifique d'une activité enzymatique différente de celle détectée par le substrat selon l'invention.

L'hydrolyse enzymatique du ou des autres substrats génère un signal détectable, différent du signal détecté par le substrat de l'invention, comme par exemple des produits colorés ou fluorescents différents, pour permettre la mise en évidence comme la détection et/ou l'identification et/ou la quantification d'un ou plusieurs microorganismes. A titre d'autre substrat spécifique, on peut utiliser tout autre substrat classiquement utilisé dans la détection des microorganismes. La concentration de l'autre substrat enzymatique spécifique est généralement comprise entre 0,01 et 1 g/l. L'homme du métier pourra déterminer facilement une telle concentration en fonction du substrat utilisé. A titre indicatif, il est possible de combiner les substrats selon l'invention avec des substrats enzymatiques de peptidase, d'osidase, d'estérase ou de réductase. En particulier, il est possible d'associer un substrat selon l'invention pour lequel P est une β-Alanine avec un substrat d'osidase, tel que le 5-Bromo-4-chloro-3-indolyl-β-glucoside, ou l'Alizarine-β-galactoside. Il est également possible d'associer un substrat selon l'invention pour lequel P est la L-Alanine avec un substrat d'estérase, tel que le 5-Bromo-6-chloro-3-indoxyl-octanoate ou le 5-Bromo-3-indoxyl-phosphate.

Selon un mode particulier de réalisation de l'invention, ledit milieu de détection et/ou d'identification selon l'invention comprend en outre au moins un autre substrat enzymatique spécifique de l'activité enzymatique détectée par le substrat selon l'invention. Par le choix particulier de substrats, il est alors possible d'identifier des groupes de microorganismes exprimant une même activité enzymatique. La concentration de l'autre substrat enzymatique spécifique est généralement comprise entre 0,01 et 1 g/l. L'homme du métier pourra déterminer facilement une telle concentration en fonction du substrat utilisé. En particulier, il est possible d'associer un substrat selon l'invention pour lequel P est une L-Alanine avec un substrat de L-Alanine aminopeptidase décrit dans la demande WO2006030119, tel que la L-Alanine-pentyl-résorufamine.

L'invention concerne également l'utilisation de substrats enzymatiques, tels que définis ci avant ou d'un milieu de détection et/ou d'identification tel que défini ci avant pour la détection chez des micro-organismes d'au moins une activité peptidase.

L'invention concerne également l'utilisation de substrats enzymatiques, tels que définis ci avant ou d'un milieu de détection et/ou d'identification tel que défini ci avant pour séparer les bactéries à Gram positif des bactéries à Gram négatif.

L'invention concerne également un procédé pour la détection chez des micro-organismes d'au moins une activité peptidase, caractérisé en ce qu'il comprend ou est constitué par les étapes suivantes:
a) disposer d'un milieu de détection et/ou d'identification tel que défini précédemment,
b) ensemencer le milieu avec un échantillon biologique à tester,
c) laisser incuber, et
d) révéler la présence d'au moins une activité peptidase

L'ensemencement des microorganismes peut être réalisé par toutes les techniques d'ensemencement connues de l'homme du métier. Une étape d'incubation peut être réalisée à une température pour laquelle l'expression de l'activité enzymatique que l'on souhaite détecter est optimale, que l'homme du métier peut choisir aisément selon l'activité enzymatique à détecter. L'étape d) peut s'effectuer par un examen visuel, par colorimétrie ou fluorimétrie. Lors de l'étape d), on peut révéler la présence de l'activité peptidase, seule ou en combinaison avec au moins une autre activité enzymatique.

L'invention concerne également un procédé pour la différenciation chez des bactéries de leur appartenance aux bactéries à Gram positif ou aux bactéries à Gram négatif, caractérisé en ce qu'il comprend ou est constitué par les étapes suivantes :
a) disposer d'un milieu de détection et/ou d'identification tel que défini précédemment,
b) ensemencer le milieu avec un échantillon biologique à tester,
c) laisser incuber, et
d) révéler la présence d'au moins une activité peptidase

Comme indiqué précédemment, l'ensemencement des microorganismes peut être réalisé par toutes les techniques d'ensemencement connues de l'homme du métier. Une étape d'incubation peut être réalisée à une température pour laquelle l'expression de l'activité enzymatique que l'on souhaite détecter est optimale, que l'homme du métier peut choisir aisément selon l'activité enzymatique à détecter. L'étape d) peut s'effectuer par un examen visuel, par colorimétrie ou fluorimétrie. Lors de l'étape d), on peut révéler la présence de l'activité peptidase, seule ou en combinaison avec d'autres activités enzymatiques.

Les exemples ci dessous sont donnés à titre explicatif et n'ont aucun caractère limitatif. Ils permettront de mieux comprendre l'invention.

### Exemple 1 - Synthèse de substrats

### • X représente S

### 1 Synthèse de 2-(2'-Aminophényl)-benzothiazole

### 1.1 2-(2'-Nitrophényl)-benzothiazole

Un mélange de 1éq de 2-Aminophénol, d'éthanol et de 1éq de 2-Nitrobenzaldéhyde a été chauffé par un bain d'huile à reflux à la température de 110°C durant une heure. Les inventeurs ont laissé refroidir le mélange à température ambiante et récupéré le précipité par filtration. Afin de récupérer un maximum de produit, le ballon a été rincé avec le filtrat pour évaporer une partie significative.

Le solide a été séché dans un dessiccateur sous pression réduite pour obtenir le produit intermédiaire.

Le solide précédemment obtenu a été dissout dans 100 ml de Dichlorométhane, puis a été ajouté petit à petit léq de 2,3-Dicyano-5,6-dichloro-parabenzoquinone à température ambiante. La réaction s'est déroulée durant une nuit, puis la verrerie a été filtrée et rincée avec du D.C.M. afin de récupérer le maximum de produit, soluble dans le D.C.M.

Le solvant a ensuite été évaporé sous pression réduite afin d'obtenir le produit.

### 1.2 2-(2'-Aminophényl)-benzothiazole

Dans un bécher de 250ml, 15g (0,15mol) d'acide polyphosphorique ont été placés, puis 4,56g (0.036mol) de 2-Aminothiophénol et 5,00g (0,036mol) d'acide anthranilique ont été ajoutés. Le mélange a été porté à 200-220°C durant 3 heures dans un bain de sable avec un bec Bunsen et remué à intervalles réguliers à l'aide d'un agitateur en verre. Le mélange a été refroidi lentement à 60°C et de la glace pilée a été ajoutée.

Ensuite, 60ml d'eau ont été ajoutés, et la solution obtenue a été basifiée à l'aide d'une solution d'hydroxyde de sodium (NaOH) jusqu'à atteindre un pH de 9-10. Un précipité orange a ainsi été obtenu, qui a été récupéré par filtration sous Buchner. Le solide obtenu a été séché puis dissout dans 50ml de Dichlorométhane, puis lavé 3 fois a l'eau. Les phases organiques ont été collectées puis séchées avec du MgSO₄, filtrées puis le solvant évaporé afin d'obtenir le produit brut. Une masse de 3,01g d'un solide jaune/vert a été obtenue après une purification par chromatographie sur colonne avec un rendement de 64%.

Les données qui ont été obtenues en RMN sont ci dessous :
¹H NMR (270 MHz, CDCl₃): δ = 6.40 (2H, broad s, Ar-N*H*₂), 6.75 (1H, d, *J* = 8 Hz, Ar-*H*), 6.78 (1H, t, *J* = 7 Hz, Ar-*H*), 7.23 (1H, t, *J* = 7 Hz, Ar-*H*), 7.35 (1H, t, *J* = 7 Hz, Ar-*H*), 7.46 (1H, t, *J* = 7 Hz, Ar-*H*), 7.71 (1H, d, *J* = 8 Hz, Ar-*H*), 7.87 (1H, d, *J* = 8 Hz, Ar-*H*), 7.97 (1H, d, *J* = 7 Hz, Ar-*H*).

### 2 Synthèse de 2-(L-Alanyl-2'-amidophényl)-benzothiazole (substrat 17)

### 2.1 Méthode des anhydrides mixés

Une masse de 1,51g (0,008 mol) de Boc-Ala-OH a été dissout dans 10ml de Tétrahydrofurane sec et un piège de CaCl₂ a été ajouté sur le col du ballon afin d'assurer l'étanchéité. Le mélange a été agité magnétiquement, et la température a été descendue à -15°C en utilisant un bain de glace congelée mélangée à des sels de NaCl. Une fois cette température atteinte, le mélange a été agité 5 minutes de plus. Puis, 1,01g (0,010 mol) de N-Méthylmorpholine a été ajouté tout en prenant bien soin que la température redescende à -15°C avant de procéder à la prochaine étape. Ensuite, 1,09g (0,008 mol) d'Isochlorobutylformate a été ajouté goutte à goutte avec la plus grande précaution, le mélange s'est troublé comme prévu à chaque goutte ajoutée.

Ensuite, 1,36g (0,006 mol) de 2-(2'-Aminophényl)-benzothiazole ont été dissous dans le minimum de T.H.F. sec, et ajouté au mélange. Le bain de glace a été retiré après 2 heures et le mélange a été agité durant la nuit.

La solution a été filtrée, le ballon a été rincé avec du T.H.F. normal et le filtrat a été conservé.

La solution a ensuite été transférée à l'aide d'une pipette pasteur dans un bécher de 400ml contenant de la glace, de l'eau ainsi que du carbonate de sodium (Na₂CO₃). La solution a ensuite été agitée durant deux heures; jusqu'à obtenir une solution limpide. La solution a ensuite été filtrée dans un petit Buchner puis le précipité a été dissous dans du méthanol afin de le recristalliser.

Une masse de 0,87g d'OF37A, une poudre beige/marron a été ainsi obtenue, avec un rendement de 37%.

Les données qui ont été obtenues en RMN sont ci dessous :
¹H NMR (270 MHz, CDCl₃): δ = 1.42 (9H, s, C-C*H*₃x3), 1.57 (3H, d, *J* = 7.92 Hz, C*H*₃-CH-), 3.50 (1H, s, N*H*-Ar), 4.52 (1H, qt ,NH-C*H*-CH₃), 4.51 (21H, d, N*H*-CH), 7.18 (1H, t, *J* = 7.42 Hz, Ar-H), 7.44 (1H, t, *J* = 7.42 Hz, Ar-H), 7.48 (1H, t, *J* = 7.42 Hz, Ar-H), 7.49 (1H, t, *J* = 7.42 Hz, Ar-H), 7.87 (1H, d, *J* = 7.92 Hz, Ar-*H*), 7.92 (1H, d, *J* = 7.92 Hz, Ar-*H*), 8.14 (1H, d, *J*=7.92 Hz, Ar-*H*), 8.82 (1H, d, *J*= 7.92 Hz, Ar-*H*).

### 2.2 Déprotection

Une masse de 0,30g (0,00075 mol) du composé à déprotéger a été placée dans un ballon équipé d'un piège à CaCl₂,et 7 ml d'acide trifluoroacétique ont été ajoutés. Le mélange a été placé sous agitation magnétique toute la nuit.

Le T.F.A. a été évaporé afin d'obtenir 0,35g d'OF38A sous forme de sel d'acide trifluoroacétique, une poudre beige/marron ayant pour rendement 88%.

Les données qui ont été obtenues en RMN sont ci dessous :
¹H NMR (270 MHz, DMSO): δ = 1.62 (3H, d, *J* = 7.42 Hz, C*H*₃-CH-), 4.35 (1H, sx, NH₂-C*H-*CH₃), 5.31 (2H, broad s, N*H*₂), 7.37 (1H, t, *J* = 7.8 Hz, Ar-*H*), 7.53 (1H, t, *J* = 7.8 Hz, Ar-*H*), 7.61 (1H, t, *J* = 7.8 Hz, Ar-*H*), 7.62 (1H, t, *J* = 7.8 Hz, Ar-*H*), 8.02 (1H, d, *J* = 7.42 Hz, Ar-*H*), 8.12 (1H, d, *J* = 7.42 Hz, Ar-*H*), 8.20 (1H, d, *J* = 7.42 Hz, Ar-*H*), 8.31 (2H, Ar-*H* + N*H*). m. p.: 150-154°C.

### 3 Synthèse de 2-(L-Alanyl-2'-amido-5'-chloro-phényl)-benzothiazole (substrat 19)

### 3.1 2-(2'-Nitro-5'-chlorophényl)-benzothiazole

Un mélange de 1,18g (0,0094mol) de 2-Aminothiophénol et de 1,74g (0,0094mol) de 5-nitro-3-chlorobenzaldéhyde dans l'éthanol (150ml) a été chauffé à reflux durant 2 heures. Après avoir été refroidie à température ambiante, la solution a été concentrée sous pression réduite. Le solide restant a été dilué dans un mélange d'eau et d'acétate d'éthyle (500ml), et la phase aqueuse a été extraite encore deux fois en utilisant de l'acétate d'éthyle (2x500ml). Les phases organiques réunies ont été séchées (MgSO₄), filtrées et concentrées afin d'obtenir le produit brut, qui a été recristallisé à partir d'éthanol. Une masse de 0,66g d'OF31A, un solide marron a été obtenue avec un rendement de 25%.

Les données qui ont été obtenues en RMN sont ci dessous :
¹H NMR (270 MHz, CDCl₃): δ = 7.55 (4H, m, Ar-*H*), 7.79 (1H, d, *J*= 2 Hz, Ar-*H*), 7.90 (1H, s, Ar-*H*), 8.10 (1H, d, *J* = δ Hz, Ar-*H*).

### 3.2 2-(2'-Amino-5'-chlorophényl)-benzothiazole

Un mélange de 0,65g (0,00223mol) de 2-(2'-Nitro-5'-chlorophényl)-benzothiazole OF31A et de 2.52g (0,01118mol) de SnCl₂, 2H₂O dans 100ml d'éthanol a été chauffé à 70°C durant 2 heures. Une étape de vérification à l'aide de chromatographie couche mince montrait que le mélange ne contenait plus aucun réactif de départ.

Après avoir laissé le mélange refroidir à température ambiante, la solution a été versée dans un bécher contenant de la glace. Le pH a été ajusté à un pH légèrement basique (pH 7-8) en ajoutant une solution aqueuse de bicarbonate de sodium (5%) NaHCO₃ avant d'avoir extrait la phase aqueuse à l'aide d'acétate d'éthyle.

La phase aqueuse ainsi obtenue a été soigneusement lavée avec de la saumure (solution aqueuse de NaCl saturée) et séchée à l'aide de sulfate de sodium afin d'obtenir 0,31 g de OF53A. un solide marron-jaune avec un rendement de 53%.

Les données qui ont été obtenues en RMN sont ci dessous :
¹H NMR (270 MHz, CDCl₃): δ = 6.41 (2H, broad s, Ar-N*H*₂), 6.74 (1H, d, *J* = 9 Hz, Ar-*H*), 7.42 (3H, m, Ar-*H*), 7.67 (1H, d, *J* = 2 Hz, Ar-*H*), 7.90 (1H, d, *J* = 8 Hz, Ar-*H*), 7.98 (1H, d, *J* = 7 Hz, Ar-*H*).

### 3.3 2-(Alanyl-2'-amido-5'-chlorophényl)-benzothiazole

### 3.3.1 Méthode des anhydrides mixés

Une masse de 0.29g (0,00153mol) de Boc-L-Ala a été dissoute dans 10ml de Tétrahydrofuranne sec. Un piège de CaCl₂ a été ajouté sur le col du ballon afin d'assurer l'étanchéité. Le mélange a été agité magnétiquement et la température du mélange a été descendue à -15°C en utilisant un bain de glace congelée mélangée à des sels de NaCl.

Une fois cette température atteinte, le mélange a été agité 5 minutes de plus. Puis, 0,19g (0,00191 mol) de N-Méthylmorpholine a été ajouté tout en prenant bien soin que la température redescende à -15°C avant de procéder à l'étape suivante. Ensuite, 0,21g (0,00153mol) d'Isochlorobutylformate a été ajouté goutte à goutte avec la plus grande précaution, le mélange s'est troublé comme prévu à chaque goutte ajoutée.

Quelques minutes plus tard, une masse de 0,30g (0,00115 mol) de 2-(2'-Amino-5'-chlorophényl)-benzothiazole a été dissoute dans le minimum de T.H.F. sec et ajouté au mélange. Le bain de glace a été retiré après 2 heures et le mélange a été laissé agiter durant la nuit.

La solution a été filtrée, et le ballon a été rincé avec du T.H.F. normal et le filtra a été conservét. La solution a été transférée à l'aide d'une pipette pasteur dans un bécher de 400 ml contenant de la glace, de l'eau ainsi que du carbonate de sodium (Na₂CO₃). Le mélange a été placé sous agitation pendant deux heures, jusqu'à obtenir une solution limpide. La solution a été filtrée dans un petit Buchner puis le précipité a été dissout dans du méthanol afin de le recristalliser.

Une masse de 0,18g d'OF55A, un solide vert clair a été obtenu avec un rendement de 36%.

Les données qui ont été obtenues en RMN sont ci dessous :
¹H NMR (270 MHz, CDCl₃): δ = 1.41 (9H, s, 3xC*H*₃), 1.56 (1H, d, *J* = 7 Hz, C*H*-CH₃), 1.58 (3H, d, *J* = 4 Hz, C*H*₃-CH), 6.45 (1H, broad s, N*H*), 6.74 (1H, d, *J* = 3 Hz, Ar-*H*), 7.45 (3H, m, Ar-*H*), 7.66 (1H, d, *J* = 3 Hz, Ar-*H*), 7.92 (2H, m, Ar-*H*).

### 3.3.2 Déprotection

Une masse de 0,18g (0,00042mol) du composé à déprotéger a été placé dans un ballon équipé d'un piège à CaCl₂, et 5ml d'acide trifluoroacétique ont été ajoutés. Le mélange a été placé sous agitation magnétique toute la nuit.

Le T.F.A. a été évaporé afin d'obtenir 0,22g d'OF57A sous forme de sel d'acide trifluoroacétique, un solide marron et collant ayant pour rendement 94%.

Les données qui ont été obtenues en RMN sont ci dessous :
¹H NMR (270 MHz, DMSO): δ = 1.50 (1H, d, *J* = 7 Hz, C*H*-CH₃), 1.55 (3H, d, *J* = 4 Hz, C*H*₃-CH), 4.80 (3H, broad s, N*H*₃), 6.87 (1H, d, *J* = 9 Hz, Ar-*H*), 7.21 (1H, d, *J* = 9 Hz, Ar-*H*), 7.52 (1H, m, Ar-*H*), 8.10 (4H, m, Ar-*H*). m. p.: 124-128°C.

### • X est O

### 1 Synthèse de 2-(2'-Aminophényl)-benzoxazole

### 1.1 2-(2'-Nitrophényl)-benzoxazole

Un mélange de 8,73g (0,0800 mol) de 2-Aminophénol, de 20 ml d'éthanol et de 12,09g (0,0800 mol) de 2-Nitrobenzaldéhyde a été chauffé par un bain d'huile à reflux à la température de 110°C durant une heure.

Le mélange a été mis à température ambiante et le précipité a été récupéré par filtration. Afin de récupérer un maximum de produit, le ballon a été rincé avec le filtrat pour évaporer une partie significative.

Le solide a été séché dans un dessicateur sous pression réduite pour obtenir 13,25g de 2-(2'-Nitro-benzylidineamino)-phénol avec un rendement de 69%.

Le solide précédemment obtenu a été dissout dans 100ml de dichlorométhane, puis a été ajouté petit à petit 12,43g (0,0547 mol) de 2,3-Dicyano-5,6-dichloroparabenzoquinone à température ambiante. La réaction a été laissée se dérouler durant la nuit, la verrerie a été filtrée puis rincée avec du D.C.M. afin de récupérer le maximum de produit.

Le solvant a ensuite été évaporé sous pression réduite afin d'obtenir 8,39g de 2-(2-Nitrophényl)-benzoxazole, un solide marron-beige avec un rendement de 64%.

Les données qui ont été obtenues en RMN sont ci dessous :
¹H NMR (270 MHz, CDCl₃): δ = 7.40 (1H, t, *J* = 5 Hz, Ar-*H*), 7.42 (1H, t, *J* = 5 Hz, Ar-*H*), 7.50 (1H, d, *J* = 7 Hz, Ar-*H*), 7.72 (1H, t, *J* = 5 Hz, Ar-*H*), 7.73 (1H, t, *J* = 5 Hz, Ar-*H*), 7.80 (1H, d, *J* = 7 Hz, Ar-*H*), 7.89 (1H, d, *J* = 7 Hz, Ar-*H*), 8.15 (1H, d, *J* = 7 Hz, Ar-*H*). m. p.: 104-107°C.

### 1.2 2-(2'-Aminophényl)-benzoxazole

Un mélange de 8g (0,033 mol) de 2-(2'-Nitrophényl)-benzoxazole et de 37,58g (0,167 mol) de SnCl₂,2H₂O dans 200ml d'éthanol a été chauffé à 70°C jusqu'à ce que la réaction soit complètement terminée.

Après avoir laissé le mélange refroidir à température ambiante, la solution a été versée dans un bécher contenant de la glace. Le pH a été ajusté à un pH légèrement basique (pH 7-8) en ajoutant une solution aqueuse de bicarbonate de sodium (5%) NaHCO₃ avant d'avoir extrait la phase aqueuse à l'aide d'acétate d'éthyle.

La phase aqueuse ainsi obtenue a été soigneusement lavée avec de la saumure (solution aqueuse de NaCl saturée) et séchée à l'aide de sulfate de sodium afin d'obtenir 4,72g de OF10C, un solide marron-jaune avec un rendement de 67%.

Les données qui ont été obtenues en RMN sont ci dessous :
¹H NMR (270 MHz, CDCl₃): δ = 6.20 (2H, broad s, Ar-N*H*₂), 6.78 (1H, t, *J* = 5 Hz, Ar-*H*), 6.81 (1H, d, *J* = 7 Hz, Ar-*H*), 7.31 (1H, t, *J* = 5 Hz, Ar-*H*), 7.32 (1H, t, *J* = 5 Hz, Ar-*H*), 7.34 (1H, t, *J* = 5 Hz, Ar-*H*), 7.58 (1H, d, *J* = 7 Hz, Ar-*H*), 7.72 (H, d, *J* = 7 Hz, Ar-*H*), 8.08 (1H, d, *J* = 7 Hz, Ar-*H*).
   m. p.: 100-104°C.

### 2 Synthèse de 2-(Glycyl-2'-amidophényl)-benzoxazole (substrat 3)

### 2.1 Méthode des anhydrides mixés

Une masse de 1,40g (0,008 mol) de N-t-Boc-Gly a été dissoute dans 10ml de tétrahydrofurane sec et un piège de CaCl₂ a été ajouté sur le col du ballon afin d'assurer l'étanchéité. Le mélange a été agité magnétiquement et la température du mélange a été descendue à -15°C en utilisant un bain de glace congelée mélangée à des sels de NaCl. Une fois cette température atteinte, le mélange a été agité 5 minutes de plus. Puis, une masse de 1,01g (0,010 mol) de N-Méthylmorpholine a été ajouté tout en prenant bien soin que la température redescende à -15°C avant de procéder à l'étape suivante. Ensuite, a été ajouté goutte à goutte avec la plus grande précaution 1,09g (0,008 mol) d'Isochlorobutylformate, le mélange s'est troublé comme prévu à chaque goutte ajoutée.

Quelques minutes plus tard, une masse de 1,26g (0,006 mol) de 2-(2'-Aminophényl)-benzoxazole a été dissoute dans le minimum de T.H.F. sec et ajouté au mélange. Le bain de glace a été retiré après 2 heures et le mélange a été placé sous agitation durant la nuit.

La solution a été filtrée, le ballon a été rincé avec du T.H.F. normal et le filtrat a été conservé. Un léger précipité était présent. La solution a été transférée à l'aide d'une pipette pasteur dans un bécher de 400ml contenant de la glace, de l'eau ainsi que du carbonate de sodium (Na₂CO₃). Le mélange a été placé sous incubation pendant 2 heures, jusqu'à obtenir une solution limpide. La solution a été filtrée dans un petit Buchner puis le précipité a été dissout dans du méthanol afin de le recristalliser.

Une masse de 0,98g d'OF27A, une poudre blanche a été obtenue avec un rendement de 45%.

Les données qui ont été obtenues en RMN sont ci dessous :
¹H NMR (270 MHz, CDCl₃): δ = 1.47 (9H, s, C-C*H*₃x3), 4.05 (1H, d, *J* = 8 Hz, -N*H*-CH₂), 4.17 (2H, d, *J* = δ Hz, CO-C*H*₂-NH), 5.38 (1H, broad s, Ar-N*H*-), 7.20 (1H, t, *J* = 5 Hz, Ar-*H*), 7.40 (1H, t, *J* = 5 Hz, Ar-*H*), 7.50 (1H, t, *J* = 5 H, Ar-*H*), 7.60 (1H, t, *J* = 5 Hz, Ar-*H*), 7.80 (1H, d, *J* = 8 Hz, Ar-*H*), 7.85 (1H, d, *J* = δ Hz, Ar-*H*), 8.25 (1H, d, *J* = 8 Hz, Ar-*H*), 8.85 (1H, d, *J* = 8 Hz, Ar-*H*).

### 2.2 Déprotection

Une masse de 0,25g (0,00068 mol) du composé à déprotéger a été placé dans un ballon équipé d'un piège à CaCl₂, et a été ajouté 7 ml d'acide trifluoroacétique. Le mélange a été placé sous agitation magnétique toute la nuit.

Le T.F.A. a été évaporé afin d'obtenir 0,29g d'OF30B sous forme de sel d'acide trifluoroacétique, une poudre beige ayant pour rendement 86%.

Les données qui ont été obtenues en RMN sont ci dessous :
¹H NMR (270 MHz, DMSO): δ = 4.05 (2H, d, *J* = 8 Hz, C*H*₂-NH₃), 4.90 (3H, broad s,N*H*₃), 7.40 (1H, t, *J* = 5 Hz, Ar-*H*), 7.46 (1H, t, *J* = 5 Hz, Ar-*H*), 7.50 (1H, d, *J* = 8 Hz, Ar-*H*), 7.70 (1H, t, *J* = 5 Hz, Ar-*H*), 7.85 (1H, t, *J* = 5 Hz, Ar-*H*), 8.20 (1H, d, *J* = 8 Hz, Ar-*H*) 8.25 (1H, d, *J* = 8Hz, Ar-*H*), 8.40 (1H, d, *J* = 8 Hz, Ar-*H*).
ESI-MS: C₁₅H₁₅N₃O₂⁺ requires *m*/*z* 269.77 [M + H]⁺, 226.96 [M + H]⁻ High-resolution M.S.E.I. for C₁₅H₁₅N₃O₂⁺. Calculated mass of molecular ion 268.1081 [M + H]⁺. Measured mass: 268.1083 [M + H]⁺.
   m. p.: 219-222°C.

### • X est NX1-CO

### 1 Synthèse de 2-(2'-Amino-5'-chloro-phényl)-quinoxazol-4-one

### 1.1 Synthèse de la 1,2-Dihydro-2-(2'-nitro-5'-chloro-phényl)-quinoxazoline-4-one (ref: VLS 276)

Une masse de 2.31g (0,017mol) de 2-Amino benzamide et 3,14g (0,017mol) de 3-Chloro-6-nitro benzaldéhyde a été chauffée dans l'éthanol à reflux durant 1 heure. Le mélange a ensuite été refroidi, et les cristaux oranges obtenus on été récoltés et séchés dans un dessiccateur à pression réduite. Une masse de 3,97g de cristaux orange clair a été obtenue avec un rendement de 76% et un point de fusion mesuré de 234-236°C.

Les données qui ont été obtenues en RMN sont ci dessous :
¹H-NMR:(DMSO) δ 6.35 (1H, t, J=2 Hz, C*H*), 6.70 (1H, d, J=1 Hz, Ph-*H*(8*H*)), 6.80 (1H, t, J=7 Hz, Ph-*H* (6*H*)), 7.05 (1H, s (broad), N*H*), 7.25 (1H, t, J=7 Hz, Ph-*H* (7*H*)), 7.65 (1H, d,d, J=6 Hz, J=1.5 Hz, Ph-*H* (5*H*)), 7.75 (1H, d,d, J=9 Hz, J=3 Hz, Ph-H (4'*H*)), 7.85 (1H, d, J=3 Hz, Ph-*H* (6'*H*)), 8.15 (1H, d, J=9 Hz, Ph-H (3'*H*)), 8.30 (1H, d (broad), N*H*-CO), High-resolution M.S.E.I for C₁₄H₁₀ClN₃O₃ Calculated mass of molecular ion 304.0483 (M+H)⁺. Measured mass: 304.0482
   υₘₐₓ cm⁻¹ 1661 (C=ONH), 1564 (NO₂), 1358 (NO₂), 1602 (C=N), 774 (C-Cl)

### 1.2 Oxydation de la 2-(2'-Nitro-5'-chloro-phényl)-quinoxazoline-4-one (ref: VLS 278)

Une masse de 9,10g (0,03 mol) de 2-(2'-Nitro-5'-chloro-phényl)-quinoxazoline-4-one a été dissoute dans 40ml d'acétone anhydre et a été traitée par 4,74g (0,03 mol) d'une solution de Permanganate de potassium dans de l'acétone anhydre. Cette solution a été ajoutée goutte à goutte au mélange sur une période de 2-3 heures à température ambiante. Ensuite la réaction a été laissée sous agitation durant la nuit. L'excès de KMnO₄ a été éliminé par l'addition de bisulfite de sodium solide en excès. Puis la solution a été filtrée et évaporée. Une masse de 2,26g d'une poudre blanche et pâle a été obtenue avec un rendement de 25% et un point de fusion mesuré de 276-278°C. Les données qui ont été obtenues en RMN sont ci dessous :
High-resolution M.S.E.I for C₁₄H₈ClN₃O₃ Calculated mass of molecular ion 302.0327 (M+H)⁺. Measured mass: 304.0327 .¹H-NMR:(DMSO) δ 7.55 (1H, t, J=7 Hz, Ph-*H* (7*H*)), 7.65 (1H, d, J=8 Hz, Ph-*H* (4'*H*)), 7.85 (1H, t, J=7 Hz, Ph-*H* (6*H*)), 7.90 (1H, d,d, J=8 Hz, J=2 Hz, Ph-*H* (5*H*)), 8.05 (1H, d, J=2 Hz, Ph-*H* (6'*H*), 8.25 (1H, d, J=8 Hz, Ph-*H* (3'*H*). υₘₐₓ cm⁻¹ 3132 (NH₂), 3070 (NH₂), 1578 (NO₂), 1368 (NO₂), 1603 (NH), 1660 (CONH), 1531 (C=N), 772 (C-Cl)

### 1.3 Synthèse de la 2-(2'-Amino-5'-chloro-phényl)-quinoxazol-4-one - Réduction du composé VLS 278 (VLS 285)

Une masse de 4,96g (0,028 mol) de SnCl₂.2H₂O a été dissoute dans un mélange de 40ml d'HCl et de 5ml d'acide acétique glacial. La solution a été portée à reflux à 120°C et 1,34g (0,0044 mol) du composé VLS 278 a été ajouté petit a petit. Le mélange a été maintenu à 120°C durant une demi-heure puis réduit à 100°C durant 2-3 heures. Un solide jaune a été obtenu et récupéré par filtration, dissous dans l'eau et rendu basique par l'addition d'une solution de 4M de NaOH. Le précipité jaune pâle a été récolté et séché avec un dessiccateur sous pression réduite. Une masse de 1,17g d'une poudre jaune a été obtenue avec un rendement de 98% et un point de fusion mesuré de 286⁰-288⁰C.

Les données qui ont été obtenues en RMN sont ci dessous :
.¹H-NMR:(OMSO) δ 6.80 (1H, d, J= 8.6 Hz, Ph-*H* (3'*H*)), 7.20 (1H, d,d, J=8.6 Hz, J=1 Hz, Ph-*H* ), 7.30 (1H, s (broad), N*H*), 7.45 (1H, t, J=8 Hz Ph-*H*), 7.70 (1H, d J=7 Hz, Ph-*H*), 7.75 (1H, t, J=7 Hz, Ph-*H*), 7.90 (1H, d J=2 Hz, Ph-*H* (6'*H*), 8.10 (1H, d,d, J=8 Hz, J=2 Hz, Ph-*H*).
   υₘₐₓ cm⁻¹ 3465 (NH₂), 3278 (NH₂), 1674 (CO), 1582 (NH), 1560 (C=N), 1159 (C-N), 766 (C-Cl)

### 2 Synthèse de 2-(L-Alanyl-2'-amido-5'-chloro-phényl)-quinoxazol-4-one (substrat 21)

### 2.1 Méthode des anhydrides mixes (VLS 286 rep)

Une masse de 0,50g (0,002mol) de tBoc-L-Ala a été dissoute dans 10ml de THF anhydre. La température a été portée à -15°C et 0,34g (0,0032mol) de NMM (*N*-Méthyle morpholine) a été ajouté sur une période de 1 minute, suivi par 0,36g (0,00266mol) d'IBCF (Isobutyle choloroformate) toujours à la même température. Apres cet ajout, le mélange a été mélangé durant 5-10 minutes puis une solution de 0.55g (0,002mol) de 2-(2'-Amino-5'-chloro-phényle)-quinoxazol-4-one dissout dans le minimum de THF anhydre, soit 5ml, a été ajoutée goutte à goutte sur une période de 2 minutes à la même température. Le mélange a été équipé d'un bain de glace durant 2-3 heures, puis laissé durant la nuit à température ambiante. Le mélange a ensuite été filtré afin d'enlever le sel de NMM, le volume du filtrat réduit afin de le verser dans un bécher contenant de l'eau et du carbonate de sodium. Le solide jaune a été récupéré et recristallisé avec de l'éthanol. Une masse de 0,43g d'une poudre jaune a été obtenue avec un rendement de 50% et un point de fusion mesuré de 268-270°C.

Les données qui ont été obtenues en RMN sont ci dessous :
¹H-NMR:(DMSO) δ 1.20 (9H, s, (C*H*₃)₃C), 1.18 (1H, s (broad), N*H*CO), 1.40 (1H, d, J=7 Hz, C*H₃*), 1.37 (1H, d, J=2 Hz, N*H*), 4.05 (1H, quartret, J=7 Hz, ala*H*), 7.25 (1H, t, J=7 Hz, N*H-*CO(C*H*₃)₃), 7.3-7.42 (2H, m, Ph-*H* ), 7.55 (1H, t, J=7 Hz, Ph-*H*), 7.65 (1H, d, Ph-*H* (3' *H*)), 8.00 (1H, d, J=7 Hz, Ph-*H* (4'*H*)), 8.60 (1H,, d, J=2 Hz, Ph-*H*), 8.65 (1H, s, Ph-*H* (6'*H*)). υₘₐₓ cm⁻¹ 1668 (CONH), 1608 (NH), 1560 (C=N), 1169 (C-N), 722 (C-Cl)

### 2.2 Déprotection (ref : VLS 289)

Le dérivé t-Boc-L-ala de la 2-(2'-Amino-5-chloro-phényle)-quinoxazoline-4-one a été déprotégé par addition d'acide trifluoro acétique. Un mélange de 0,30g (0,0007 mol) de 2-(t-Boc-L-ala-2'-amido-5-chloro-phényle)-quinoxazoline-4-one et de 5ml d'acide trifluoro acétique a été mélangé durant la nuit à température ambiante. L'excès d'ATF a été évaporé et le solide marron ainsi obtenu a été séché à l'aide d'un dessicateur sous pression réduite. Une masse de 0,26g (0,00057 mol) d'un solide marron orangé a été obtenue avec un rendement de 81% et un point de fusion mesuré de 268-270°C.

Les données qui ont été obtenues en RMN sont ci dessous :
¹H-NMR:(DMSO) δ 1.40 (3H, d, C*H*₃), 3.40 (1H, quartret, *H*-ala), 7.55 (1H, t, J=7 Hz, Ph-*H*), 7.67 (111, dd, J=8 Hz, J=2 Hz, Ph-*H*), 7.71 (1H, d, J=8 Hz, Ph-*H*), 7.83 (1H, m, Ph-*H* ), 7.90 (1H, d, J=8 Hz, Ph-*H*), 8.20 (1H, d,d, J=8 Hz, J=1 Hz, Ph-*H*), 8.30 (2H, s (broad), N*H*₂), 10.92 (1H, s (broad), N*H*CO). υₘₐ cm⁻¹ 1667 (CONH), 1605 (NH), 1560 (C=N), 1435 (CH₃), 1181 (C-N), 770 (C-Cl)

### • X est NX1

### 1 Synthèse de 2-(2'-Amino-4',5'-cyclomethylenedioxy-phenyl)-3-phenethylbenzimidazole (ou 2-(2'-Amino-4',5'-méthylènedioxy-phényl)-3-phényléthylbenzimidazole

### 1.1 Synthèse du 2-(2'-Nitro-4',5'-cyclomethyleneddioxy-phenyl)-3-phenylethyl-benzimidazole (ou 2-(2'-Nitro-4',5'-méthylènedioxy-phényl)-3-phényléthyl-bcnzimidazole

### 1.1.1 N-Phényléthyl-2-nitroaniline (ref : VLS 209)

Une masse de 4.8g (0.04 mol) de 2-Phényléthylamine a été ajoutée goutte à goutte, et ce à température ambiante, à un mélange de 5,6g (0,04 mol) de 2-Fluoronitrobenzène et de 11.06g (0,08 mol) de carbonate de potassium anhydre dans 30ml de DMSO. Le mélange a été chauffé à 100°C durant 3 heures. Ensuite le mélange a été refroidi à température ambiante et a été ajouté à 100ml d'eau, ce qui a conduit à la précipitation d'un composé orange clair. Le solide a été récupéré par filtration et séché à l'aide d'un dessiccateur à pression réduite. Une masse de 9,4g de cristaux orange a été obtenue avec un rendement de 98%. Le point de fusion trouvé a été cohérent avec la valeur théorique, soit 64-66⁰C.

Les données qui ont été obtenues en RMN sont ci dessous :
¹H-NMR: (CDCl₃) δ 3.00 (2H, t, J=7 Hz, -C*H*₂-), δ 3.55 (2H, t, J=7 Hz, -C*H*₂-), δ 6.65 (1H, m, Ph-*H*), δ 6.85 (1H, dd, J=8 Hz, J=2 Hz, Ph-*H*), δ 7.20-7.50 (6H, m, Ph-*H*), δ 8.10 (1H, s(broad), N*H*), δ 8.20 (1H, dd, J=8 Hz, J=2 Hz, Ph-*H*).

### 1.1.2 N-Phényléthyl-1,2-diaminobenzène (ref: VLS 210)

A une suspension de 0,52g (0,002 mol) d'acétonate d'acétyle de cuivre dans l'éthanol, une solution de 0,38g (0,01 mol) de borohydrure de sodium a été ajoutée, agitée magnétiquement sous Azote à température ambiante. A cette solution ont été ajoutés 2,42g (0,01 mol) du composé nitro VLS209 dans l'éthanol suivi de 0,76g (0,02 mol) de borohydrure de sodium dans l'éthanol. Le mélange a été laissé à agiter durant la nuit sous Azote à température ambiante. Ensuite le mélange a été concentré afin de retirer l'excès d'éthanol et de l'eau a été ajoutée. Puis une étape d'extraction a été réalisée avec 2x20ml de DCM et la phase organique a été lavée plusieurs fois avec de l'eau, séchée à l'aide de carbonate de potassium et concentrée à l'évaporateur rotatif. Une masse de 1,69g d'un liquide sombre et huileux qui s'est solidifié par la suite a été obtenue, avec un rendement de 80%. Le point de fusion trouvé a été cohérent avec la valeur théorique, soit 42-44°C.

Les données qui ont été obtenues en RMN sont ci dessous :
¹H-NMR: (CDCl₃) δ 2.95 (2H, t, J=7 Hz, -C*H*₂-), δ 3.27 (2H, s (broad), N*H*₂), δ 3.38 (2H, t, J=7 Hz, -C*H*₂), δ 6.69 (3H, m, Ph-*H*), δ 6.82 (1H, m, Ph-*H*), δ 7.10-7.40 (5H, m, Ph-*H*).

### 1.1.3 2-(2'-Nitro-4',5'-cyclomethylenedioxy-phenyl)-3-phenethylbenzimidazole (ref: VLS 213) (ou 2-(2'-Nitro-4',5'-méthylènedioxy-phényl)-3-phényléthyl-benzimidazole

A une solution sous agitation magnétique de 5,08g (0,024 mol) de *N*-Phényléthyl-1,2-diaminobenzène, de 4,8g (0,024 mol) de 6-Nitropiperonal dans 25ml de DMF et d'1ml d'eau, 8,60g (0,014 mol) d'oxone (monopersulphate de potassium) ont été ajoutés durant une période de 15 minutes à température ambiante. Le mélange a été refroidi par un bain d'eau car la réaction était exothermique, et laissé à agiter durant la nuit. Ensuite de l'eau a été ajoutée au mélange et deux extractions consécutives au DCM ont été réalisées. Les phases organiques réunies ont été lavées à l'eau plusieurs fois, séchées (MgSO₄) et concentrées pour donner le produit brut d'une couleur jaune/marron. Une recristallisation avec de l'éthanol a été réalisée afin de purifier le composé. Une masse de 6,19g de cristaux marron a été obtenue avec un rendement de 67%, et un point de fusion mesuré de 162-164°C.

Les données qui ont été obtenues en RMN sont ci dessous :
High-resolution M.S.E.I for C₂₂H₁₇N₃O₄ Calculated mass of molecular ion 388.1292 (M+H)⁺. Measured mass: 388.1297 .¹H-NMR: (CDCl₃) δ 3.10 (2H, t, J=7 Hz,-C*H*₂-), δ 4.20 (2H, t, J=7 Hz,-C*H*₂-), δ 6.02 (1H, s, Ph-*H*), δ 6.18 (2H, s, O-C*H*₂-O), δ 6.80 (2H, dd, J=8 Hz, J=2 Hz, Ph-*H*), δ 7.10-7.40 (5H, m, Ph-*H*), δ 7.48 (1H, m, Ph-*H*), δ 7.65 (1H, s, Ph-*H*), δ 7.80 (1H, m, Ph-*H*).
   υₘₐₓ cm⁻¹ 1364 (NO₂), 1523 (NO₂), 1610 (C=N), 1152 (C-N), 1207 (C-N), 1089 (C-O), 1473 (C-H), 728 (-CH₂).

### 1.2 Synthèse du 2-(2'-Amino-4',5'-cyclomethylenedioxy-phenyl))-3-phenylethyl-benzimidazole (ou 2-(2'-Amino-4',5'-méthylènedioxyphényl))-3-phényléthyl-benzimidazole

A une suspension de 0,16g (0,0006 mol) d'acétonate d'acétyle de cuivre dans l'éthanol, une solution de 0,11g (0,003 mol) de borohydrure de sodium a été ajoutée, agitée magnétiquement sous Azote à température ambiante. A cette solution a été ajouté 1,07g (0,003 mol) du composé nitro VLS213 dans l'éthanol suivi de 0,23g (0,006 mol) de borohydrure de sodium dans l'éthanol. Le mélange a été laissé à agiter durant la nuit sous Azote à température ambiante. Ensuite le mélange a été concentré afin de retirer l'excès d'éthanol et de l'eau a été ajoutée. Puis, une extraction a été réalisée avec 2x20ml de DCM et la phase organique a été lavée plusieurs fois avec de l'eau, séchée (K₂CO₃) et concentrée à l'évaporateur rotatif. Une masse de 0,92g d'une poudre marron a été obtenue avec un rendement de 87% et un point de fusion mesuré de 170-172⁰C.

Les données qui ont été obtenues en RMN sont ci dessous :
High-resolution M.S.E.I for C₂₂H₁₉N₃O₂ Calculated mass of molecular ion 358.1550 (M+H)⁺. Measured mass: 358.1550
   ¹H-NMR: (CDCl₃) δ 3.00 (2H, t, J=7 Hz -C*H*₂-), δ 4.40 (2H, t, J=7 Hz, -C*H*₂), δ 6.00 (2H, s, O-C*H*₂-O), δ 6.35 (1H, s, Ph-*H* (6'H), δ 6.50 (1H, s, Ph-*H* (3' H)), δ 7.00 (2H, m, Ph-*H*), δ 7.20-7.40 (5H, m, Ph-*H*), δ 7.55 (1H, m, Ph-*H*), δ 7.85 (1H, m, Ph-*H*).
   υₘₐₓ cm⁻¹ 3434 (N-H), 3350 (N-H), 1618 (C=N), 1219 (C-O), 1037 (C-N), 1157 (C-N), 1495 (Ar), 1473 (Ar), 1521 (Ar), 728 (-CH₂-)

### 2 Synthèse de 2-(L-Alanyl-2'-amido-4',5'-cyclomethylenedioxy-phenyl)-3-phenethyl-benzimidazole (substrat 16) (ou 2-(L-Alanyl-2'-amido-4',5'-méthylènedioxy-phényl)-3-phényléthyl-benzimidazole)

Une masse de 0,50g (0,0026 mol) de tBoc-L-alanine a été dissoute dans 10ml de DCM anhydre et 0,54g (0,0026 mol) de Dicyclohexylcarbodiimide a été ajouté à la solution sous agitation, suivi par 0,35g (0,0026 mol) d'hydroxybenzatriazole. Le mélange a été laissé sous agitation à température ambiante durant 45 minutes puis 0,65g (0,0019 mol) de 2-(2'-Amino-4',5'-methylenedioxolanephenyl)-3-phényléthyl-benzimidazole (ou 2-(2'-Amino-4',5'-méthylènedioxyphényl)-3-phényléthyl-benzimidazole) a été ajouté avant de laisser poursuivre la réaction de nuit. Le lendemain, le mélange a été filtré à travers de la celite et le solvant évaporé, puis le liquide résultant a été versé dans un mélange eau/glace. Le précipité huileux ainsi formé a été récupéré, dissout dans de l'acétate d'éthyle et lavé plusieurs fois avec une solution de 10% de NaHCO₃ puis avec de l'eau. La phase organique a été séchée (MgSO₄) et concentrée. Le solide marron ainsi obtenu a été déprotégé par l'addition d'une solution saturée de HCl dans de l'acétate d'éthyle. Le précipité a été agité avec une solution aqueuse saturée en Na₂CO₃ et 10ml de DCM. La phase organique a été séparée et séchée (MgSO₄) puis concentrée. Une masse de 0,37g d'un solide marron a été obtenue avec un rendement de 38% et un point de fusion mesuré de 80-82°C.

Le composé a été purifié avec une chromatographie sur colonne (gel de silice), ce qui a donné un solide jaune/marron.

Les données qui ont été obtenues en RMN sont ci dessous :
¹H-NMR: (CDCl₃) δ 1.30 (3H, d, J=6 Hz, ala C*H*₃), 1.50 (2H, s (broad), N*H*₂), 3.07 (2H, t, J=7 Hz, C*H*₂), 3.40 (1H, quartret, ala *H*), 4.45 (2H, t, J=7 Hz, C*H*₂), 6.04 (2H, s, C*H*₂O₂), 6.58 (1H, s, Ph-*H* (6'*H*)), 6.85 (2H, m, Ph-*H* (6*H*,7*H*)), 7.15 (3H, m, Ph-*H*), 7.35 (2H, d,d, Ph-*H* (5*H*, 8*H*)), 8.10 (1H, s, Ph-*H* (3'*H*)), υₘₐₓ cm⁻¹1667 (C=O), 1622 (C=N), 1239 (C-O), 1152 (C-N), 728 (-CH₂-)

### 3 Synthèse de 2-(L-Alanyl-2'-amidophényl)-3-phényléthyl-benzimidazole (substrat 14, = VLS237)

2-(2'-Nitrophényl)-3-phényléthyl-benzimidazole : Ce substrat a été synthétisé de façon analogue à la synthèse du 2-(2'-Nitro-4',5'-méthylènedioxyphényl)-3-phényléthyl-benzimidazole. Un rendement de 78% de cristaux blancs a été obtenu, et un point de fusion mesuré de 128-130 °C. Les données qui ont été obtenues en RMN sont ci dessous:
High-resolution M.S.E.I for C₂₁H₁₇N₃O₂. Calculated mass of molecular ion 344.1394 (M+H)⁺. Measured masse: 344.1394. ¹H-NMR: (CDCl₃) δ 3.07 (2H, t, J=6.93 Hz, -C*H*₂-), 4.20 (2H, t, J=6.93 Hz, -C*H*₂-N), 6.79 (2H, dd, J=7.67 and 1.24 Hz, Ph-*H*), 6.85 (1H, dd, J=7.42 and 1.73 Hz, Ph-*H*), 7.17 (3H, m, Ph-*H*), 7.36 (2H, m, Ph-*H*), 7.47 (1H, m, Ph-*H*), 7.53 (1H, t, J=7.42 Hz, Ph-*H*), 7.63 (1H, t, J=7.92 Hz, Ph-*H*), 7.81 (1H, m, Ph-*H*), 8.17 (1H, dd, J=8.16 and 1.48 Hz, Ph-*H*). IR: υₘₐₓ cm⁻¹ 1520 (NO₂), 1358 (NO₂), 1454 (C=N), 1155 (C-N), 746 (-CH₂-).

2-(2'-Aminophényl)-3-phényléthyl-benzimidazole: Ce substrat a été synthétisé de façon analogue à la synthèse du 2-(2'-Amino-4',5'-méthylènedioxyphényl)-3-phényléthyl-bcnzimidazole (page 27, line 31). Un rendement de 77% de poudre marron a été obtenu, et un point de fusion mesuré de 130-132 °C. Les données qui ont été obtenues en RMN sont ci dessous:
High-resolution M.S.E.I for C₂₁H₁₉N₃. Calculated mass of molecular ion 314.1652 (M+H)⁺. Measured mass: 314.1652. ¹H-NMR: (CDCl₃) δ 3.10 (2H, t, J=7.92 Hz, -C*H*₂-), δ 4.40 (2H, t, J=7.92 Hz, -C*H*₂-), δ 4.65 (2H, s (broad), N*H*₂), δ 6.80-7.90 (13H, m, Ph-*H*) IR: υₘₐₓ cm⁻¹ 3417 (-NH₂), 3400 (NH₂), 1616 (C=N), 1484 (C-H), 1454 (C-H), 1155 (C-N), 733 (CH₂), 698 (CH₂), 1591 (Ph), 1484 (Ph).

2-(L-Alanyl-2'-amidophenyl)-3-phényléthyl-benzimidazole: Ce substrat a été synthétisé de façon analogue à la synthèse du 2-(L-Alanyl-2'-amido-4',5'-méthylènedioxyphenyl)-3-phényléthyl-benzimidazole (page 28, line 19). Un rendement de 58% de poudre jaune/marron a été obtenu, et un point de fusion mesuré de 80-82 °C. Les données qui ont été obtenues en RMN sont ci dessous:
High-resolution M.S.E.I for C₂₅H₂₄N₄O₃. Calculated mass of molecular ion 429.1921 (M+H). Measured mass: 429.1926. ¹H-NMR: (CDCl₃) δ 1.30 (3H, d, J=6 Hz, ala C*H*₃), 1.50 (2H. s (broad), N*H*₂), 3.07 (2H, t, J=7 Hz, C*H*₂ ), 3.40 (1H, q, J=6 Hz, ala-*H*), 4.45 (2H, t, J=7 *Hz*, -C*H*₂-), 6.04 (2H, s, -C*H*₂O₂-), 6.58 (1H, s, C6'-*H*), 6.85 (2H, m, C6-*H*, and C7-*H*), 7.15 (3H, m, Ph-*H*), 7.35 (2H, dd, J=7 Hz, J=2 Hz, C5-*H*, C8-*H*), 8.10 (1H, s, N-*H*), IR υₘₐₓ cm⁻¹ 2918 (-CH₂-), 2850 (CH₃), 1675 (C=O),1625 (NH₂), 1622 (C=N), 1152 (C-N), 749 (-CH₂-), 1476 (Ar), 1455 (Ar), 1034 (C-N), 1365 (CH₃).

### Exemple 2 - Utilisation de substrats de formule I selon l'invention pour détecter une activité peptidase

### a) Synthèse des substrats

- Substrat No 1 - 2-(L-Alanyl-2'-amidophényl)-benzoxazole - VLS206 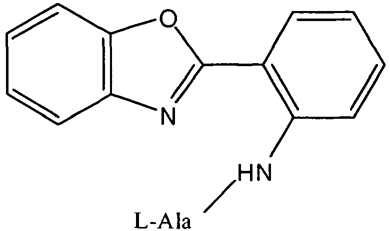
- Substrat No 2 - 2-(β-Alanyl-2'-amidophényl)-benzoxazole - OF22A 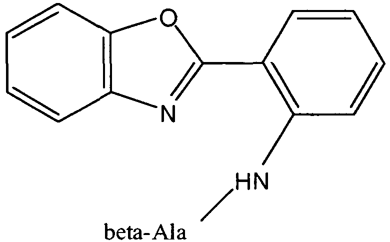
- Substrat No 3 - 2-(Glycyl-2'-amidophényl)-benzoxazole - OF30B 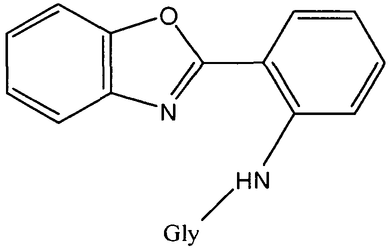
- Substrat No 4 - 2-(L-Alanyl-2'-amidophényl)-5-chloro-benzoxazole - 35 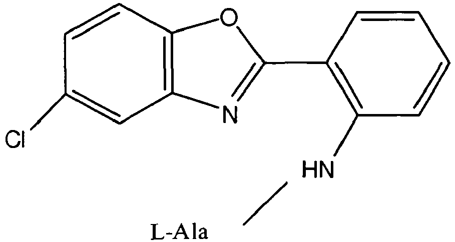
- Substrat No 11 - 2-(β-Alanyl-2'-amido-4',5'-dimethoxy-phényl)-benzoxazole - OF24A 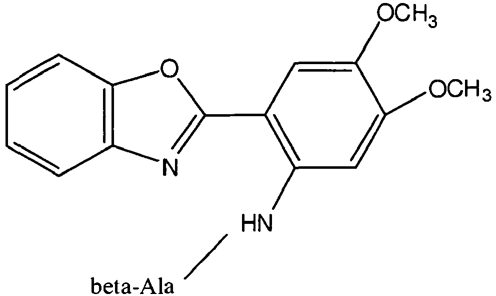
- Substrat No 17 - 2-(L-Alanyl-2'-amidophényl)-benzothiazole - OF38A 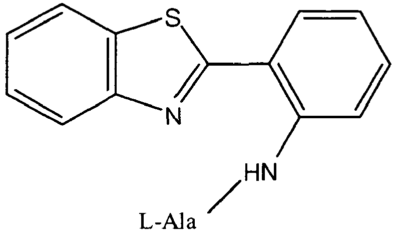
- Substrat No 18 - 2-(β-Alanyl-2'-amidophényl)-benzothiazole - OF33A 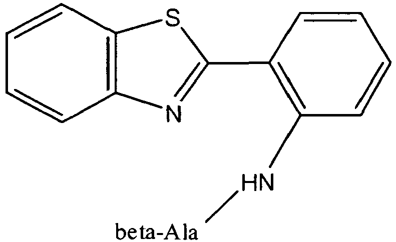
- Substrat No 19 - 2-(L-Alanyl-2'-amido-5'-chloro-phényl)-benzothiazole 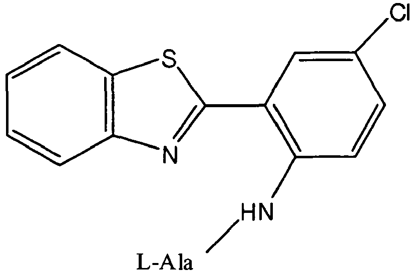
- Substrat No 16 - 2-(L-Alanyl-2'-amido 4',5' methylenedioxolane) 3phényléthyl-benzimidazole (ou 2-(L-Alanyl-2'-amido 4',5'-méthylènedioxyphényl) 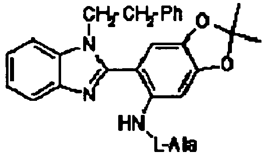
- Substrat No 14 -2-(L-Alanyl-2'-amidophényl)-3-phényléthyl-benzimidazole (= VLS237)

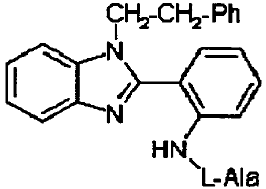

Les substrats No 1, 2, 4 et 11 ont été synthétisés de façon analogue à la synthèse du 2-(Glycyl-2'-Amidophényl)-benzoxazole (substrat No 3) dans l'exemple 1.

De même les substrats No 17 et 18 ont été synthétisés de façon analogue à la synthèse du 2-(L-Alanyl-2'-amido-5'-chloro-phényl)-benzothiazole (substrat No 19) dans l'exemple 1.

### b) Préparation du milieu

40 mg de chaque substrat sont dissous dans 4ml de Diméthyl formamide (DMF). Cette solution est intégralement reprise dans 400ml de gélose Columbia préalablement autoclavée et maintenue en surfusion à 50°C. La concentration finale pour chaque substrat est donc de 100mg/l.

Chacun des milieux est réparti en boîte de Petri de 90mm de diamètre à raison de 20ml par boîte.

### c) Ensemencement et incubation

18 souches de micro-organismes issues de collections et appartenant à différentes espèces de bactéries et de levures sont ensemencées sur ces milieux par isolement semi-quantitatif de 10µl d'une suspension à 0,5 McFarland diluée au 20^{e}. Les milieux sont incubés 24 heures à 37°C, puis les colonies formées sont examinées visuellement sous éclairage UV à 360-365nm.

### d) Lecture des résultats

Les résultats obtenus sont présentés dans le tableau 2 et le tableau 3.

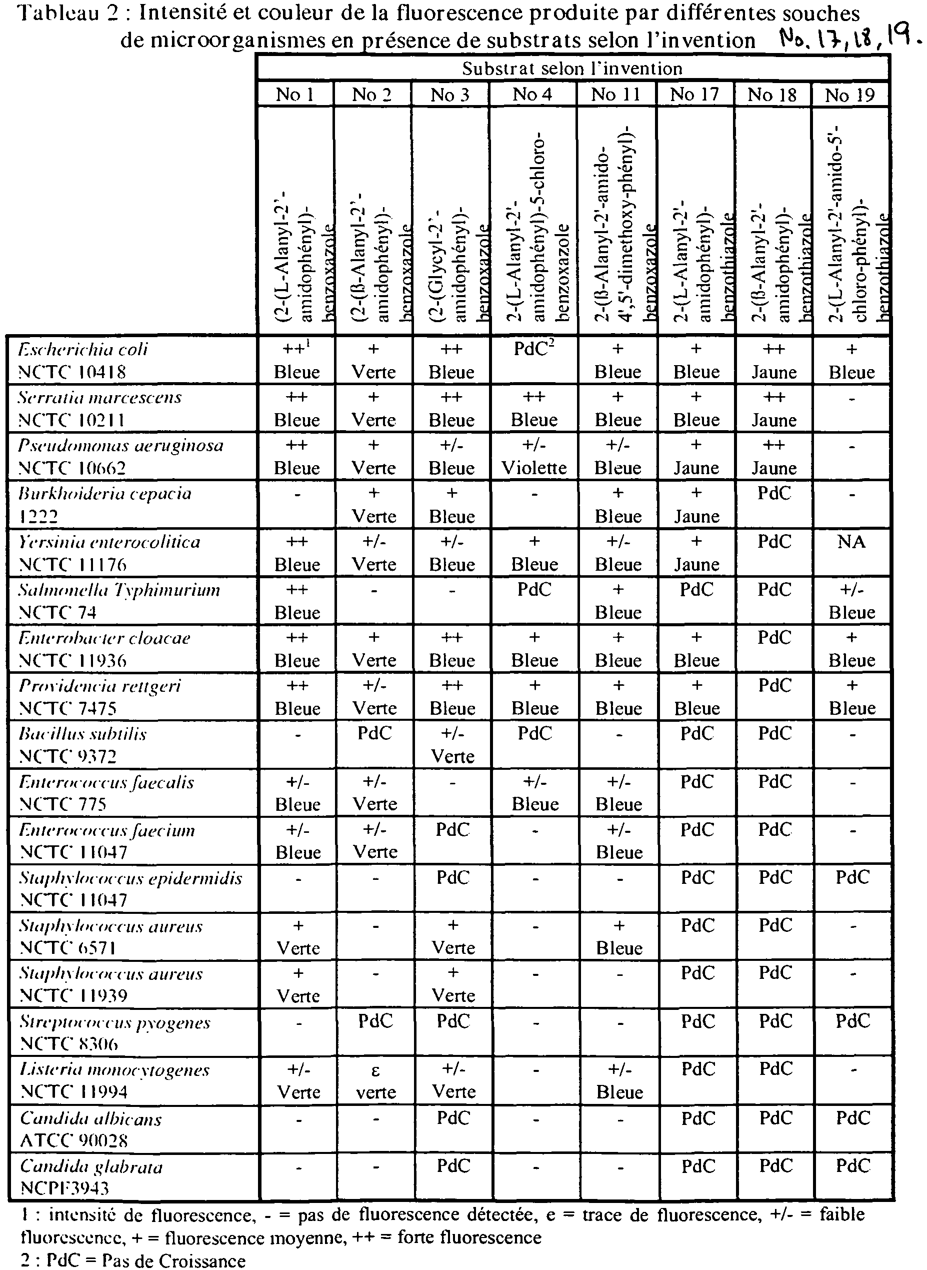

**Tableau 3 : Croissance, couleur et fluorescence produite par différentes souches de microorganismes en présence de substrats**

| | Substrat 16 | | Substrat 14 | |
|---|---|---|---|---|
| | Cr | F | Cr | F |
| *Escherichia coli* NCTC 10418 | + | ++ Bleue | + | - |
| *Serratia marcescens* NCTC 10211 | + | ++ Bleue | + | +/- Pourpre |
| *Pseudomonas aeruginosa* NCTC 10662 | + | - | + | - |
| *Burkholderia cepacia* 1222 | + | ++ Bleue | + | - |
| *Yersinia enterocolitica* NCTC 11176 | + | - | + | - |
| *Salmonella typhimurium* NCTC 74 | ++ | ++ Bleue | ++ | - |
| *Citrobacter freundii* 46262 (wild) | + | ++ Bleue | + | - |
| *Morganella morqanii* 462403 (wild) | + | + Bleue | + | - |
| *Enterobacter cloacae* NCTC 11936 | + | ++ Bleue | + | +/- Pourpre |
| *Providencia rettgeri* NCTC 7475 | + | ++ Bleue | + | - |
| | | | | |
| *Bacillus subtilis* NCTC 9372 | + | - | + | - |
| *Enterococcus faecails* NCTC 775 | + | - | + | - |
| *Enterococcus faecium* NCTC 7171 | + | - | + | - |
| *Staphylococcus epidermidis* NCTC 11047 | + | - | + | - |
| *Staphylococcus aureus* NCTC 6571 | + | - | + | - |
| MRSA NCTC 11939 | + | - | + | - |
| *Streptococcus pyogenes* NCTC 8306 | + | - | + | - |
| *Listeria monocytogenes* NCTC 11994 | + | - | + | - |
| *Candida albicans* ATCC 90028 | + | - | + | - |
| *Candida glabrata* NCPF 3943 | +/- | - | +/- | - |

| | | | | |
|---|---|---|---|---|
| Cr : croisssance - Co : couleur - F : fluorescence | | | | |

Contrairement à ce qui est observé avec des substrats à base de 7-Amino-4-méthyl-coumarine (AMC) telle que la L-Alanine-7-AMC, pour tous les substrats selon l'invention testés, la fluorescence était localisée au niveau de la colonie uniquement ou à proximité immédiate.

Pour les substrats 1, 2 et 3 pour lesquels seul P diffère (L-Alanine, ß-Alanine et Glycine respectivement), l'intensité et/ou la couleur de la fluorescence produite par une même souche différaient. Avec le substrat 1, l'ensemble des souches de bactéries à Gram négatif (à l'exception de la souche de *Burkholderia cepacia*) produisaient une forte fluorescence bleue. Les deux souches d'entérocoques produisaient une faible fluorescence bleue et les deux souches de *Staphylococcus aureus* produisaient une fluorescence verte moyenne. En revanche la souche de *B. cepacia* produisait une fluorescence bleue moyenne avec le substrat 3 et verte avec le substrat 2.

Ces résultats indiquaient également que la couleur de la fluorescence, son intensité et la toxicité variaient lorsque P est constant mais que X et/ou R2 variaient. C'était le cas entre les substrats 1, 4, 17 et 19. Ainsi avec le substrat 4, la souche de *Pseudomonas aeruginosa* produisait une fluorescence violette faible et pour les souches de *S. aureus* aucune fluorescence n'était détectée. Le substrat 17 inhibait toutes les souches de bactéries à Gram positif et les levures, et avec le substrat 19, la souche de *P. aeruginosa* était négative.

De même, il y a de grandes différences de couleur de fluorescence (de longueur d'onde d'émission), d'intensité et de toxicité entre les substrats 2, 11 et 18 pour lesquels P représente la ß-Alanine.

### c) Conclusion

En fonction de l'application recherchée, il convient de définir P, X, R2 ou R1.

Par exemple, le substrat 1 (2-(L-Alanyl-2'-amidophényl)-benzoxazole) permet de différencier les bactéries à Gram négatif (fluorescence bleue intense), des levures et des bactéries à Gram positif, et, parmi ces dernières, de différencier les entérocoques (fluorescence bleue faible) et les *S. aureus* (fluorescence verte moyenne) des autres. Le substrat 3 permet également de distinguer les bactéries à Gram négatif (fluorescence bleue) des autres microorganismes.

Le substrat 19 permet de séparer certaines entérobactéries (*Eschenichia coli, Salmonella, Enterobacten, Monganella* et *Providencia*) des autres microorganismes.. D'autres substituants, notamment l'utilisation d'autres peptides peuvent permettre de séparer d'autres groupes de microorganismes. En fonction des groupements X, R1 et R2, il est possible de faire varier tant la couleur que l'intensité de la fluorescence, la sensibilité ou la spécificité de la détection de groupes de microorganismes ou même la toxicité. Ces variations sont très utiles notamment dans le cas de combinaisons avec d'autres substrats enzymatiques.

## Revendications

1. Substrat enzymatique pour la détection d'une activité peptidase de formule (I) suivante : 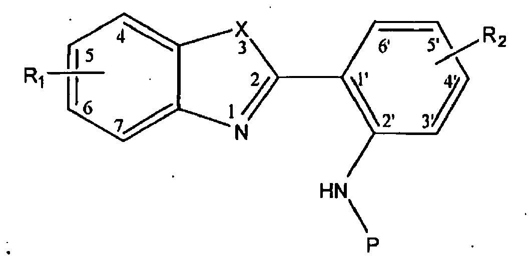 selon laquelle :
□ X est S
□ R1 est rien, ou un des substituants suivants : Cl, Br, F, I, OH, un groupement alkyle, aryle, carboxyle,
□ R2 est rien ou un des substituants suivants : Cl ; O-CH₃ ; F, diéthylènediamine-CH₃, NR3R4, Br, I, OH, un groupement alkyle, aryle, carboxyle, NO₂ 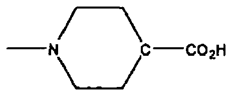 ou O-CH₂-O ; dans ce dernier cas, R2 représente un cycle accolé à l'aminophényl et deux de leurs sommets sont partagés ;
□ R3 et R4 sont indépendamment H ou un groupement alkyle ayant de 1 à 4 atomes de carbone
□ P est une chaine peptidique comprenant de 1 à 10 acides aminés et ne comprenant pas un agent de blocage en son extrémité N-terminale.

2. Substrat enzymatique selon la revendication 1 selon lequel :
• X est S
• R1 n'est rien
• R2 n'est rien
• P est une chaine peptidique comprenant de 1 à 10 acides aminés et ne comprenant pas un agent de blocage en son extrémité N-terminale.

3. Substrat enzymatique selon la revendication 1 selon lequel:
• X est S
• R1 n'est rien
• R2 est CI
• P est une chaine peptidique comprenant de 1 à 10 acides aminés et ne comprenant pas un agent de blocage en son extrémité N-terminale.

4. Milieu réactionnel comprenant au moins un substrat enzymatique de formule (I) suivante : 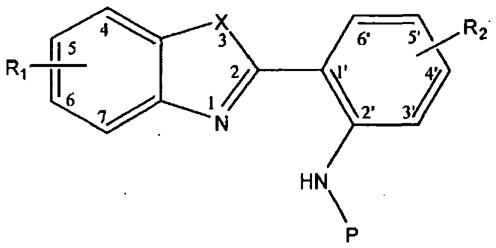 selon laquelle :
□ X est S
□ R1 est rien, ou un des substituants suivants : Cl, Br, F, I, OH, un groupement alkyle, aryle, carboxyle,
□ R2 est rien ou un des substituants suivants : Cl ; O-CH₃ ; F, diéthylènediamine-CH₃, NR3R4, Br, I, OH, un groupement alkyle, aryle, carboxyle, NO₂ 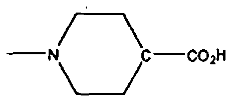 ou O-CH₂-O ; dans ce dernier cas, R2 représente un cycle accolé à l'aminophényl et deux de leurs sommets sont partagés ;
□ R3 et R4 sont indépendamment H ou un groupement alkyle ayant de 1 à 4 atomes de carbone
□ P est une chaine peptidique comprenant de 1 à 10 acides aminés.

5. Milieu de détection et/ou d'identification de microorganismes comprenant au moins un substrat enzymatique de formule (I) suivante : 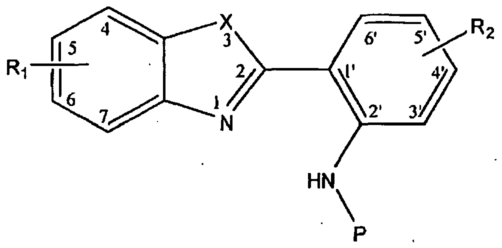 selon laquelle :
□ X est S
□ R1 est rien, ou un des substituants suivants : Cl, Br, F, I, OH, un groupement alkyle, aryle, carboxyle,
□ R2 est rien ou un des substituants suivants : Cl ; O-CH₃ ; F, diéthylènediamine-CH₃, NR3R4, Br, I, OH, un groupement alkyle, aryle, carboxyle, NO₂ 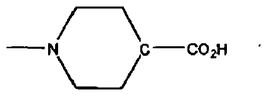 ou O-CH₂-O ; dans ce dernier cas, R2 représente un cycle accolé à l'aminophényl et deux de leurs sommets sont partagés ;
□ R3 et R4 sont indépendamment H ou un groupement alkyle ayant de 1 à 4 atomes de carbone
□ P est une chaine peptidique comprenant de 1 à 10 acides aminés.

6. Milieu de détection selon la revendication 5 comprenant en outre au moins un autre substrat enzymatique, spécifique d'une activité enzymatique différente de celle détectée par le substrat par le substrat de formule (I) compris dans le milieu selon la revendication 5.

7. Milieu de détection selon la revendication 5 ou 6 comprenant en outre au moins un autre substrat enzymatique spécifique de l'activité enzymatique détectée par le substrat de formule (I) compris dans le milieu selon la revendication 5 ou 6.

8. Utilisation des substrats enzymatiques, tels que définis dans l'une quelconque des revendications 1 à 3 ou d'un milieu de détection et/ou d'identification selon l'une quelconque des revendications 5 à 7 pour la détection chez des micro-organismes d'au moins une activité peptidase.

9. Procédé pour la détection chez des micro-organismes d'au moins une activité peptidase, **caractérisé en ce qu'**il comprend les étapes suivantes :
a) disposer d'un milieu de détection et/ou d'identification selon l'une quelconque des revendications 5 à 7,
b) ensemencer le milieu avec un échantillon biologique à tester,
c) laisser incuber, et
d) révéler la présence d'au moins une activité peptidase

## Patentansprüche

1. Enzymsubstrat zum Nachweisen einer Peptidaseaktivität der folgenden Formel (I): 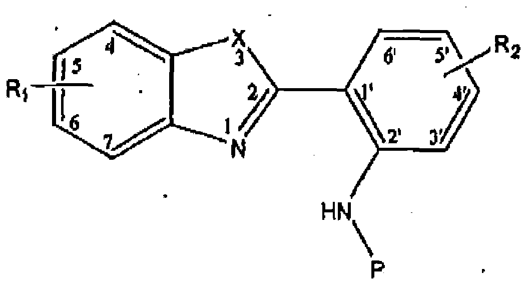 in der
□ X S bedeutet
□ R1 nichts oder einen der folgenden Substituenten bedeutet: Cl, Br, F, I, OH, eine Alkyl-, Aryl-, Carboxylgruppe,
□ R2 nichts oder einen der folgenden Substituenten bedeutet: Cl; O-CH₃; F, Diethylendiamin-CH₃, NR3R4, Br, I, OH, eine Alkyl-, Aryl-, Carboxylgruppe, NO₂ 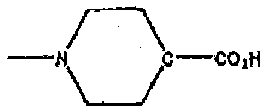 oder O-CH₂-O; wobei im letzten Fall R2 einen mit dem Aminophenyl fusionierten Zyklus bedeutet und zwei ihrer Ringglieder geteilt sind;
□ R3 und R4 unabhängig H oder eine Alkylgruppe mit 1 bis 4 Kohlenstoffatomen bedeuten
□ P eine Peptidkette mit 1 bis 10 Aminosäuren bedeutet, die an ihrem N-terminalen Ende kein Blockierungsmittel umfasst.

2. Enzymsubstrat nach Anspruch 1, wobei
• X S bedeutet
• R1 nichts bedeutet
• R2 nichts bedeutet
• P eine Peptidkette mit 1 bis 10 Aminosäuren bedeutet, die an ihrem N-terminalen Ende kein Blockierungsmittel umfasst.

3. Enzymsubstrat nach Anspruch 1, wobei
• X S bedeutet
• R1 nichts bedeutet
• R2 Cl bedeutet
• P eine Peptidkette mit 1 bis 10 Aminosäuren bedeutet, die an ihrem N-terminalen Ende kein Blockierungsmittel umfasst.

4. Reaktionsmedium, umfassend mindestens ein Enzymsubstrat der folgenden Formel (I): 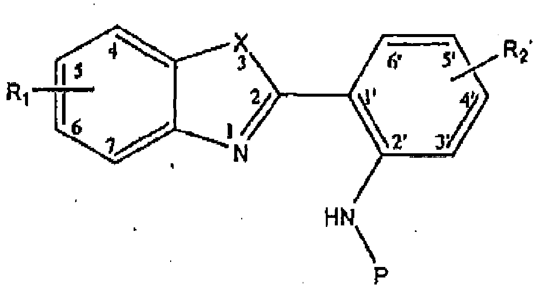 in der
□ X S bedeutet
□ R1 nichts oder einen der folgenden Substituenten bedeutet: Cl, Br, F, I, OH, eine Alkyl-, Aryl-, Carboxylgruppe,
□ R2 nichts oder einen der folgenden Substituenten bedeutet: Cl; O-CH₃; F, Diethylendiamin-CH₃, NR3R4, Br, I, OH, eine Alkyl-, Aryl-, Carboxylgruppe, NO₂ 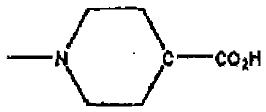 oder O-CH₂-O; wobei im letzten Fall R2 einen mit dem Aminophenyl fusionierten Zyklus bedeutet und zwei ihrer Ringglieder geteilt sind;
□ R3 und R4 unabhängig H oder eine Alkylgruppe mit 1 bis 4 Kohlenstoffatomen bedeuten
□ P eine Peptidkette mit 1 bis 10 Aminosäuren bedeutet.

5. Medium zum Nachweisen und/oder Identifizieren von Mikroorganismen, das mindestens ein Enzymsubstrat der folgenden Formel (I): 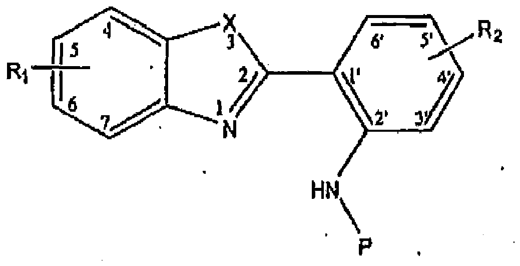 in der
□ X S bedeutet
□ R1 nichts oder einen der folgenden Substituenten bedeutet: Cl, Br, F, I, OH, eine Alkyl-, Aryl-, Carboxylgruppe,
□ R2 nichts oder einen der folgenden Substituenten bedeutet: Cl; O-CH₃; F, Diethylendiamin-CH₃, NR3R4, Br, I, OH, eine Alkyl-, Aryl-, Carboxylgruppe, NO₂ 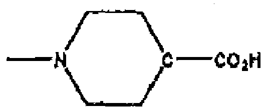 oder O-CH₂-O; wobei im letzten Fall R2 einen mit dem Aminophenyl fusionierten Zyklus bedeutet und zwei ihrer Ringglieder geteilt sind;
□ R3 und R4 unabhängig H oder eine Alkylgruppe mit 1 bis 4 Kohlenstoffatomen bedeuten
□ P eine Peptidkette mit 1 bis 10 Aminosäuren bedeutet,
umfasst.

6. Nachweismedium nach Anspruch 5, das weiterhin mindestens ein anderes Enzymsubstrat umfasst, das für eine Enzymaktivität spezifisch ist, die sich von derjenigen, die von dem Substrat der Formel (I), das in dem Medium nach Anspruch 5 enthalten ist, nachgewiesen wird, unterscheidet.

7. Nachweismedium nach Anspruch 5 oder 6, das weiterhin mindestens ein anderes Enzymsubstrat umfasst, das für die Enzymaktivität spezifisch ist, die von dem Substrat der Formel (I), das in dem Medium nach Anspruch 5 oder 6 enthalten ist, nachgewiesen wird.

8. Verwendung von Enzymsubstraten wie in einem der Ansprüche 1 bis 3 definiert oder eines Nachweis- und/oder Identifikationsmediums nach einem der Ansprüche 5 bis 7 zum Nachweisen von mindestens einer Peptidaseaktivität bei Mikroorganismen.

9. Verfahren zum Nachweisen von mindestens einer Peptidaseaktivität bei Mikroorganismen, **dadurch gekennzeichnet, dass** es die folgenden Schritte umfasst:
a) Verfügen über ein Nachweis- und/oder Identifikationsmedium nach einem der Ansprüche 5 bis 7,
b) Inokulieren des Mediums mit einer zu testenden biologischen Probe,
c) Inkubierenlassen und
d) Anzeigen des Vorhandenseins von mindestens einer Peptidaseaktivität.

## Claims

1. An enzyme substrate for detecting peptidase activity, of formula (I) below: 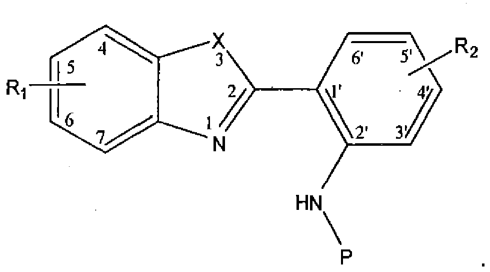 in which:
□ X is S;
□ R1 is nothing or one of the following substituents: Cl, Br, F, I, OH, or an alkyl, aryl or carboxyl group;
□ R2 is nothing or one of the following substituents: Cl; O-CH₃; F, diethylenediamine-CH₃, NR3R4, Br, I, OH, an alkyl, aryl or carboxyl group, NO₂, 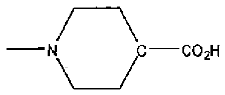 or O-CH₂-O; in this last case, R2 represents a ring fused to the aminophenyl and two of their ring members being shared;
□ R3 and R4 are independently H or an alkyl group having from 1 to 4 carbon atoms;
□ P is a peptide chain comprising from 1 to 10 amino acids and not comprising a blocking agent at its N-terminal end.

2. The enzyme substrate as claimed in claim 1, in which:
• X is S;
• R1 is nothing;
• R2 is nothing;
• P is a peptide chain comprising from 1 to 10 amino acids and not comprising a blocking agent at its N-terminal end.

3. The enzyme substrate as claimed in claim 1, in which:
• X is S;
• R1 is nothing;
• R2 is Cl;
• P is a peptide chain comprising from 1 to 10 amino acids and not comprising a blocking agent at its N-terminal end.

4. A reaction medium comprising at least one enzyme substrate of formula (I) below: 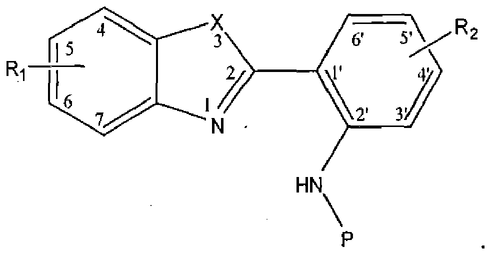 in which:
□ X is S;
□ R1 is nothing or one of the following substituents: Cl, Br, F, I, OH, or an alkyl, aryl or carboxyl group;
□ R2 is nothing or one of the following substituents: Cl; O-CH₂-O; O-CH₃; F, diethylenediamine-CH₃, NR3R4, Br, I, OH, an alkyl, aryl or carboxyl group, NO₂, 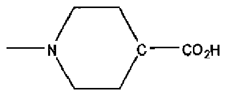 or O-CH₂-O; in this last case, R2 represents a ring fused to the aminophenyl and two of their ring members being shared;
□ R3 and R4 are independently H or an alkyl group having from 1 to 4 carbon atoms;
□ P is a peptide chain comprising from 1 to 10 amino acids.

5. A reaction medium comprising at least one enzyme substrate of formula (I) below: 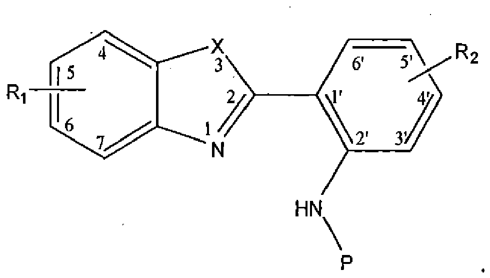 in which:
□ X is S;
□ R1 is nothing or one of the following substituents: CI, Br, F, I, OH, or an alkyl, aryl or carboxyl group;
□ R2 is nothing or one of the following substituents: Cl; O-CH₃; F, diethylenediamine-CH₃, NR3R4, Br, I, OH, an alkyl, aryl or carboxyl group, NO₂, 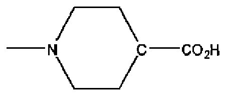 or O-CH₂-O; in this last case, R2 represents a ring fused to the aminophenyl and two of their ring members being shared;
□ R3 and R4 are independently H or an alkyl group having from 1 to 4 carbon atoms;
□ P is a peptide chain comprising from 1 to 10 amino acids.

6. The reaction medium according to claim 5, further comprinsing at least one other enzyme substrate, specific for an enzymatic activity different to the one detected by the substrate of formula (I), included in the medium according to claim 5.

7. The reaction medium according to claim 5 or 6, further comprinsing at least one other enzyme substrate, specific for the enzymatic activity detected by the substrate of formula (I), included in the medium according to claim 5.

8. A use of enzyme substrates as defined in any one of claims 1 to 3 or a detection and/or identification medium according to any one of claims 5 to 7, for detecting at least one peptidase activity in microorganisms.

9. A method for detecting at least one peptidase activity in microorganisms, **characterized in that** it comprises the following steps:
a) providing a detection and/or identification medium as claimed in any one of claims 5 to 7,
b) inoculating the medium with a biological sample to be tested,
c) leaving this to incubate, and
d) revealing the presence of at least one peptidase activity.
